(19) **Europäisches Patentamt**
**European Patent Office**
**Office européen des brevets**

(11) **EP 3 915 471 B1**

(12) **EUROPEAN PATENT SPECIFICATION**

(45) Date of publication and mention
of the grant of the patent:
**13.11.2024  Bulletin 2024/46**

(21) Application number: **20744616.2**

(22) Date of filing: **15.01.2020**

(51) International Patent Classification (IPC):
***A61B 5/026*** (2006.01)      ***A61B 5/0285*** (2006.01)

(52) Cooperative Patent Classification (CPC):
**A61B 5/0261; A61B 5/1176;** A61B 5/02405;
A61B 5/11; A61B 5/14532; A61B 5/18;
A61B 5/4845; A61B 5/4866

(86) International application number:
**PCT/JP2020/001076**

(87) International publication number:
**WO 2020/153194 (30.07.2020 Gazette 2020/31)**

(54) **INFORMATION PROCESSING DEVICE, INFORMATION PROCESSING METHOD, AND PROGRAM**

INFORMATIONSVERARBEITUNGSVORRICHTUNG,
INFORMATIONSVERARBEITUNGSVERFAHREN UND PROGRAMM

DISPOSITIF DE TRAITEMENT D'INFORMATIONS, PROCÉDÉ DE TRAITEMENT
D'INFORMATIONS ET PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **23.01.2019  JP 2019009024**

(43) Date of publication of application:
**01.12.2021  Bulletin 2021/48**

(73) Proprietor: **Sony Group Corporation
108-0075 Tokyo (JP)**

(72) Inventors:
• **OKUBO, Atsushi
  Tokyo 108-0075 (JP)**

• **WAKITA, Yoshihiro
  Tokyo 108-0075 (JP)**

(74) Representative: **MFG Patentanwälte
Meyer-Wildhagen Meggle-Freund
Gerhard PartG mbB
Amalienstraße 62
80799 München (DE)**

(56) References cited:
**EP-A1- 3 175 786        JP-A- 2008 284 165
JP-A- 2014 184 002        JP-A- 2014 184 002
JP-A- 2014 194 765        JP-A- 2015 191 268
JP-A- 2015 191 268        JP-A- 2017 144 225
US-A1- 2017 231 544       US-A1- 2018 153 422**

**Description**

Field

The present disclosure relates to an information processing device, an information processing method, and a program.

Background

A blood flowmeter can be attached to a person to be measured and easily measure blood flow information (biological information) such as a pulse and a blood flow rate without inflicting discomfort, pain, and the like on the person to be measured. For example, examples of the blood flowmeter include Patent Literature 1 described below. Further, by analyzing a change with time of blood flow information of a specific part of the body of the person to be measured acquired by such a blood flowmeer, it is possible to acquire various kinds of information concerning a state of the body of the person to be measured useful for the person to be measured and the like. The specific part means a region of the body of the person to be measured where the blood flow information can be suitably acquired and specifically means a region where blood vessels are concentratedly present such as a cheek, a fingertip, or a palm.

Citation List

Patent Literature

Patent Literature 1: JP 2018-68428 A

Document US 2017/0231544 A1 discloses a biological information detection device that includes: a light source for irradiating a test portion of a subject, a light detector for detecting light received from the test portion, and a calculating circuit for generating a signal of blood flow information in a target area of the test portion based on the received light. The calculating circuit is further configured to detect a magnitude of inclination and orientation of the test portion by image recognition and determines the target area according to the magnitude of the inclination and orientation of the test portion. Document US 2018/0153422 A1 discloses a further biological information detection apparatus,

Summary of the invention

Technical Problem

In the blood flowmeter of patent literature 1, by attaching and fixing the blood flowmeter to a part of the body of the person to be measured, it is possible to continuously acquire blood flow information (biological information) of a specific part. However, the attaching such a blood flowmeter sometimes hinders the motion of the person to be measured. Further, with the blood flowmeter, even in a part where blood flow information can be suitably acquired because blood vessels are concentratedly present, it is difficult to acquire the blood flow information if the part is a part where it is difficult to attach the blood flowmeter such as a cheek or the like of the person to be measured.

On the other hand, there is also a blood flowmeter that can acquire blood flow information of a person to be measured even if the blood flowmeter is not attached to a part of the body of the person to be measured (e.g. the above-mentioned US documents) . However, since such a blood flowmeter is not fixed to the body of the person to be measured, it is difficult to accurately acquire blood flow information of a specific part every time. In such a case, since the blood flow information of the specific part cannot be continuously or intermittently acquired, even if a change with time of the blood flow information is analyzed, it is difficult to acquire various kinds of information concerning a state of the body of the person to be measured.

Therefore, in view of the circumstances, the present invention proposes an information processing device, an information processing method, and a program capable of easily acquiring biological information of a specific part of the body of a person to be measured without attaching the device.

Solution to Problem

According to a first aspect, the present invention provides an information processing device according to independent claim 1. According to a second aspect, the present invention provides an information processing method according to independent claim 11. According to a third aspect, the present invention provides a program according to independent claim 12. Further aspects of the present invention are set forth in the dependent claims, the drawings and the following description.

Summary of the disclosure

According to the present disclosure, an information processing device is provided that includes: an irradiating unit that irradiates an organism with coherent light; a light receiving unit that receives the light reflected on the organism; and a calculating unit that calculates, based on sensing data acquired from the light receiving unit, biological information linked with position information concerning a position of the reflection of the light in the organism.

Moreover, according to the present disclosure, an information processing method is provided that includes: irradiating an organism with coherent light; receiving the light reflected on the organism; and calculating, based on sensing data based on the received light, biological information linked with position information concerning a position of the reflection of the light in the organism.

Moreover, according to the present disclosure, a program is provided that causes a computer to realize: a function of controlling an irradiating unit to irradiate an organism with coherent light; a function of controlling a light receiving unit to receive the light reflected on the organism; and a function of calculating, based on sensing data acquired from the light receiving unit, biological information linked with position information concerning a position of the reflection of the light in the organism.

Brief Description of the Drawings

FIG. 1 is an explanatory diagram (No. 1) for explaining a blood flow measuring method applied to an embodiment of the present disclosure.

FIG. 2 is an explanatory diagram (No. 2) for explaining the blood flow measuring method applied to the embodiment of the present disclosure.

FIG. 3 is an explanatory diagram for explaining a first processing method applied to the embodiment of the present disclosure.

FIG. 4 is an explanatory diagram for explaining a second processing method applied to the embodiment of the present disclosure.

FIG. 5 is an explanatory diagram for explaining an example of the configuration of an information processing system 10 according to a first embodiment of the present disclosure.

FIG. 6 is a block diagram illustrating an example of the configuration of a biological information sensor 100 according to the first embodiment of the present disclosure.

FIG. 7 is a block diagram illustrating an example of the configuration of a camera 200 according to the first embodiment of the present disclosure.

FIG. 8 is a block diagram illustrating an example of the configuration of a server 300 according to the first embodiment of the present disclosure.

FIG. 9 is a flowchart illustrating an example of an information processing method according to the first embodiment of the present disclosure.

FIG. 10 is an explanatory diagram for explaining an example of the configuration of an information processing system 10a according to a second embodiment of the present disclosure.

FIG. 11 is a block diagram illustrating an example of the configuration of a biological information sensor 100a according to the second embodiment of the present disclosure.

FIG. 12 is an explanatory diagram for explaining a principle of a method of calculating depth information.

FIG. 13 is an explanatory diagram for explaining a method of calculating depth information using the biological information sensor 100a according to the second embodiment of the present disclosure.

FIG. 14 is an explanatory diagram for explaining an example of an image 804 based on the depth information according to the second embodiment of the present disclosure.

FIG. 15 is a block diagram illustrating an example of the configuration of the server 300a according to the second embodiment of the present disclosure.

FIG. 16 is a flowchart (No. 1) illustrating an example of an information processing method according to the second embodiment of the present disclosure.

FIG. 17 is a flowchart (No. 2) illustrating an example of the information processing method according to the second embodiment of the present disclosure.

FIG. 18 is a flowchart (No. 3) illustrating an example of the information processing method according to the second embodiment of the present disclosure.

FIG. 19 is an explanatory diagram for explaining an example of the image 804 and a detection signal 800 based on depth information according to the second embodiment of the present disclosure.

FIG. 20 is an explanatory diagram for explaining an example of an irradiation pattern and a sampling pattern according to a modification of the first and second embodiments of the present disclosure.

FIG. 21 is an explanatory diagram for explaining an example 1 according to the second embodiment of the present disclosure.

FIG. 22 is an explanatory diagram for explaining an example 2 according to the second embodiment of the present disclosure.

FIG. 23 is an explanatory diagram (No. 1) for explaining an example 3 according to the second embodiment of the present disclosure.

FIG. 24 is an explanatory diagram (No. 2) for explaining the example 3 according to the second embodiment of the present disclosure.

FIG. 25 is a block diagram illustrating an example of a hardware configuration of an information processing device 900 according to an embodiment of the present disclosure.

Description of Embodiments

Preferred embodiments of the present disclosure are explained in detail below with reference to the accompanying drawings. Note that, in this specification and the drawings, redundant explanation about constituent elements having substantially the same functional configurations is omitted by denoting the constituent elements with the same reference numerals and signs.

In this specification and the drawings, similar constituent elements in different embodiments are sometimes distinguished by adding different alphabets after the same reference number. However, when it is not particularly necessary to distinguish each of similar constituent elements, only the same reference number is added.

Note that, in the following explanation, a target person of measurement whose biological information (for example, blood flow information) is measured using a biological information sensor according to an embodiment of the present disclosure explained below is referred to as person to be measured. In the following explanation, a person using an information processing system according to the embodiment of the present disclosure explained below is referred to as user. The measuring person is also included in the user.

Note that the explanation is made in the following order.

1. Background until creation of an embodiment of the present disclosure
1.1. Background
1.2. About a blood flow information measuring method
2. First Embodiment
2.1. Configuration of an information processing system 10 according to a first embodiment
2.2. Configuration of a biological information sensor 100 according to the first embodiment
2.3. Configuration of a camera 200 according to the first embodiment
2.4. Configuration of a server 300 according to the first embodiment
2.5. Information processing method according to the first embodiment.
3. Second Embodiment
3.1. Configuration of an information processing system 10a according to a second embodiment
3.2. Configuration of a biological information sensor 100a according to the second embodiment
3.3. Configuration of a server 300a according to the second embodiment
3.4. Information processing method according to the second embodiment
3.5. Modification
4. Examples according to the embodiments of the present disclosure
4.1. Example 1
4.2. Example 2
4.3. Example 3
5. Summary
6. About a hardware configuration
7. Supplementation

<<1. Background until creation of an embodiment of the present disclosure>>

<1.1. Background>

First, before details of an embodiment of the present disclosure are explained, a background until the present inventors created the embodiment of the present disclosure is explained.

As explained above, by analyzing a change with time of acquired blood flow information (biological information) of a specific part of the body of a person to be measured, it is possible to acquire various kinds of state information (information such as presence or absence of a disease) concerning the state of the body of the person to be measured useful for the person to be measured and the like. In order to acquire highly accurate state information, it is preferable that a change with time of blood flow information, which is an analysis target, is continuously or intermittently acquired from a specific part that can be measured in a preferred state. Note that the specific part is a region of the body of the person to be measured where blood flow information can be suitably acquired because blood vessels are concentratedly present such as a cheek, a fingertip, or a palm.

Therefore, there is a device that can continuously acquire blood flow information of a specific part by being attached and fixed to a part of the body of a person to be measured. However, the attaching such a device sometimes hinders a motion of the person to be measured. Further, with the device, even in a part where blood flow information can be suitably acquired because blood vessels are concentratedly present, it is difficult to acquire the blood flow information if the part is a part where it is difficult to attach the device such as a cheek or the like of the person to be measured.

On the other hand, there is also a device that can acquire blood flow information of a person to be measured without being attached to a part of the body of the person to be measured. However, since such a device is not fixed to the body of the person to be measured, it is difficult to accurately acquire blood flow information of a specific part every time. In such a case, since the blood flow information of the specific part cannot be continuously or intermittently acquired, even if a change with time of the blood flow information is analyzed, it is difficult to acquire highly accurate state information.

Therefore, in view of such circumstances, the present inventors created the embodiment of the present disclosure capable of easily acquiring blood flow information of a specific part of the body of a person to be measured without being attached. Details of the embodiment of the present disclosure created by the present inventors are explained below.

Incidentally, in the embodiment of the present disclosure, blood flow information concerning a blood flow of a person to be measured is acquired as an example of biological information. Specifically, the blood flow information means information concerning a blood flow such as a heart rate, a pulse, an average blood flow rate, a blood flow amount, and a speed distribution of particles in blood vessels. The pulse rate means the number of times per unit time of beats of an artery appearing on a body surface of the like when a change in pressure occurs on an artery inner wall because blood is sent to the entire body through the artery by contraction (beat) at a constant rhythm of the muscle of the heart. Further, the blood flow rate means a rate of blood (blood components) flowing in one or a plurality of blood vessels in a measurement region, which is a measurement target. The blood flow amount means a blood amount that passes, in a unit time, the one or the plurality of blood vessels in the measurement region. The speed distribution of particles in a blood vessel means a speed distribution of density of particles staying or flowing in a blood vessel such as red blood cells in the one or the plurality of blood vessels in the measurement region. Blood can be considered a mixed body of substances having a plurality of flow rates. Characteristics of a blood flow can also be indicated by movements of blood cells, which are particles in a blood vessel, that is, a flow rate of the blood cells. The flow rate of the blood cells can be used as an important indicator indicating the characteristics of the blood flow. In the present disclosure, an average moving rate of the blood cells (the particles) in the blood vessel is referred to as average blood flow rate.

<1.2. About a blood flow information measuring method>

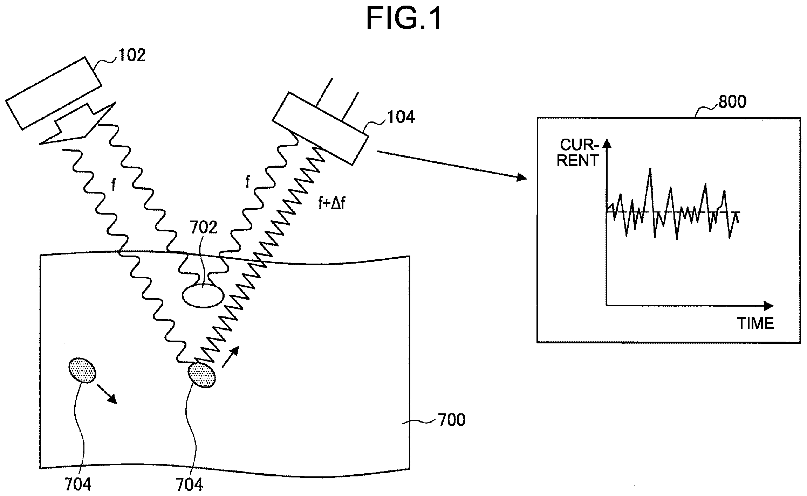

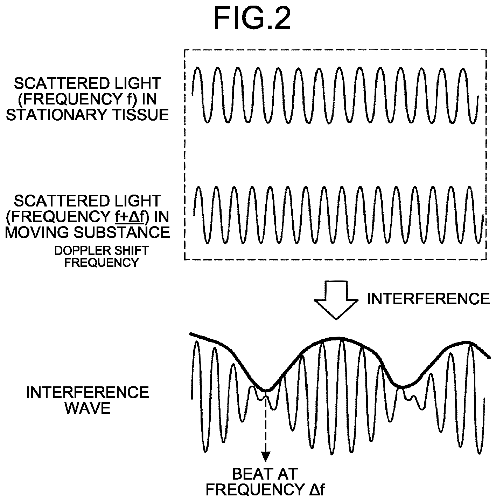

A measuring method for blood flow information in the embodiment of the present disclosure is explained. Examples of a blood flow information measuring method according to this embodiment include analysis techniques for a speed distribution using a laser doppler blood flow measuring method and a Dynamic light scattering (DLS) method. First, an interference phenomenon of coherent light by a blood flow common to both of the laser doppler blood flow measuring method and the DLS method is explained with reference to FIG. 1 and FIG. 2. FIG. 1 and FIG. 2 are explanatory diagrams for explaining a blood flow measuring method applied to the embodiment of the present disclosure. Specifically, FIG. 1 and FIG. 2 schematically illustrate an interference phenomenon of coherent light by a blood flow. A reference numeral 800 in FIG. 1 indicates an example of a waveform of a detection signal obtained by the measurement.

The blood flow information measuring method according to the embodiment of the present disclosure is a method making use of the fact that, for example, as illustrated in FIG. 1, when a measurement region (a part of the body) 700 of a person to be measured (an organism) is irradiated with light from an irradiating unit 102 of a biological information sensor 100 explained below, light scattered by scattering substances (mainly red blood cells) moving in a blood vessels of the person to be measured causes interference light through a position movement of the scattering substances. In the blood flow information measuring method, the interference light is received by a light receiving unit 104 of the biological information sensor 100. Blood flow information can be calculated from a distribution of a Doppler shift frequency and speckle contrast in the received interference light.

Specifically, as illustrated in FIG. 1, when light having a frequency f with which the measurement region 700 of the person to be measured is irradiated by the irradiating unit 102 is scattered by a stationary tissue 702 such as a skin or a subcutaneous tissue of the person to be measured, scattered light of the light maintains the frequency f. On the other hand, when the light having the frequency f with which the measurement region 700 of the person to be measured is scattered by a moving substance (for example, a red blood cell; the red blood cell is a substance having a diameter of 8 to 10 μm) 704 moving in the blood vessel of the person to be measured, scattered light of the light is frequency-shifted by a position movement of the moving substance 704 and has a frequency f+Δf. As illustrated in FIG. 2, since the scattered light having the frequency f scattered by the stationary tissue 702 and the scattered light having the frequency f+Δf scattered by the moving substance 704 interfere, the light receiving unit 104 can detect interference light having an optical beat. Note that, in general, the shift frequency Δf is extremely smaller than the frequency f of the irradiation light.

It is possible to obtain blood flow information illustrated as a reference numeral 800 in FIG. 1 by processing interference light (a detection signal) detected by the light receiving unit 104. Note that, since the detection signal 800 is a superimposed signal of optical beats having a plurality of different frequencies by scattered light from the moving substance 704 in a plurality of blood vessels performing different motions, the detection signal is seen as an irregular signal like white noise as illustrated in FIG. 1. However, since the detection signal 800 is the superimposed signal of the interference beats having the plurality of frequencies as explained above, by performing frequency analytical processing explained below on the detection signal, it is possible to acquire speed distribution information of a particle motion that causes a frequency shift, in other words, a Doppler shift. Therefore, in the laser doppler blood flow measuring method, it is possible to acquire blood flow information such as an average blood flow rate by grasping a speed distribution of the moving substance 704 such as a red blood cell in a blood flow.

Alternatively, when the measurement region 700 of the person to be measured is irradiated with coherent light, the scattered light by the scattering substance in the measurement region 700 interferes with the coherent light, whereby a pattern called speckle pattern can be obtained. The speckle pattern causes a change by being scattered by the moving substance 704. Therefore, in the DLS method, it is possible to obtain blood flow information by calculating, based on information concerning fluctuation in the speckle pattern obtained by processing the interference light (the detection signal) detected by the light receiving unit 104, speed information of the moving substance 704 such as a red blood cell in a blood flow.

With the blood flow information measuring method, it is possible to acquire blood flow information in a biological tissue in a range in which irradiated light reaches. Therefore, it is also possible to acquire blood flow information in a region including not only blood vessels on the surface of the skin of the person to be measured but also blood vessels in deep parts at depth of a certain degree from the skins in the entire body of the person to be measured.

Further, in the embodiment of the present disclosure, the blood flow information is acquired by processing the detection signal 800 detected by the light receiving unit 104 as explained above. In the embodiment of the present disclosure, for example, two methods explained below can be used as a method of processing for the detection signal 800 for acquiring blood flow information. Specifically, examples of a processing method in this embodiment include, in general, a first processing method for performing frequency analysis processing (Fourier transform) first used in the laser doppler speed detection and a second processing method for calculating an autocorrelation function used in the DLS method. Details of the first and second processing methods are explained below in order.

First processing method

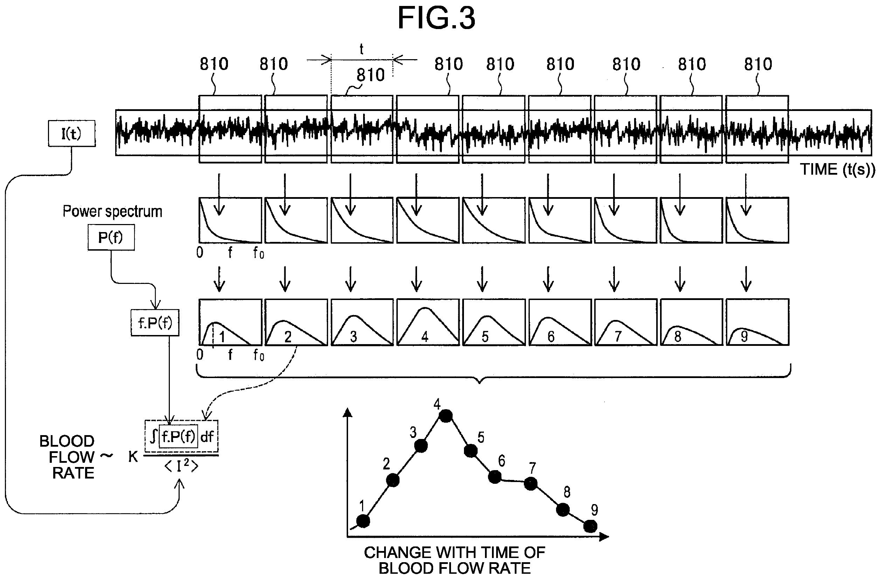

First, the first processing method is explained with reference to FIG. 3. FIG. 3 is an explanatory diagram for explaining the first processing method applied to this embodiment. In the first processing method, first, as illustrated in FIG. 3, blood flow information is acquired by performing, for each plurality of ranges (windows 810 in FIG. 3), frequency analysis processing such as Fast Fourier Transform (FFT) on a detection signal (I(t) in FIG. 3) obtained by the light receiving unit 104.

Specifically, in the first processing method, the FFT is performed on the detection signal in each predetermined time range to acquire a plurality of power spectra (P(f) in FIG. 3), which are functions of frequencies. Further, it is possible to obtain an average blood flow rate by calculating, for each frequency, products of the acquired power spectra with a beat frequency in a proportional relation with speed (fP(f) in FIG. 3) and performing integration in the entire region of the power spectra to standardize the power spectra. It is possible to acquire a waveform indicating a change with time of the average blood flow rate by acquiring a plurality of blood flow rates based on the plurality of power spectra calculated from the plurality of windows 810 in different time ranges. Further, in the first processing method, by performing interpolation processing or the like on the acquired waveform, it is possible to acquire a pulse waveform (not illustrated) indicating a change in the blood flow rate due to a pulse and calculate a pulse rate and the like from the pulse waveform. Note that, in FIG. 3, the plurality of windows 810 for deciding a range for power spectra generation are not superimposed one another and displayed. However, in actual processing, the windows 810 can be superimposed one another. A plurality of power spectrum rows arranged along a time series may be more precisely generated by performing processing for each of the superimposed windows 810.

Second processing method

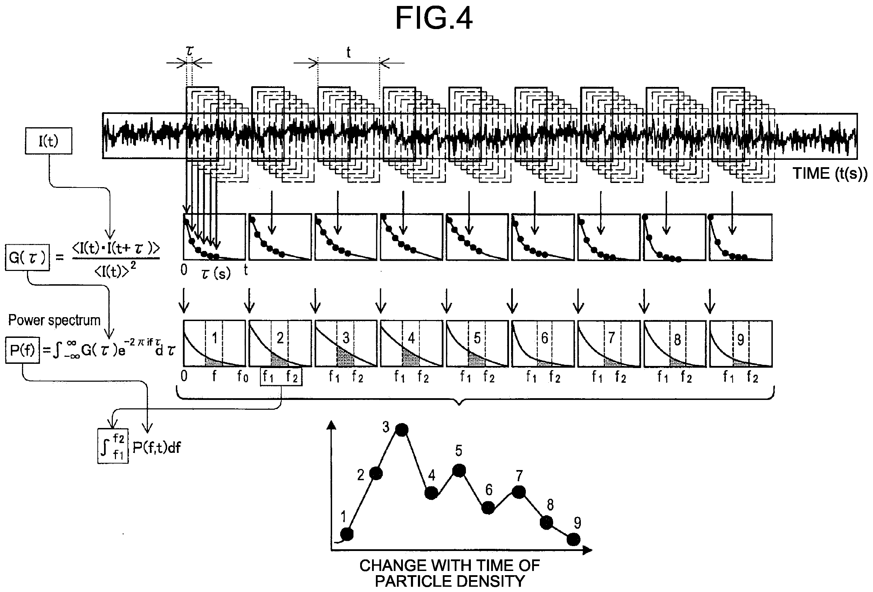

The second processing method is explained with reference to FIG. 4. FIG. 4 is an explanatory diagram for explaining the second processing method applied to this embodiment. In the second processing method, first, as illustrated in FIG. 4, blood flow information is acquired by calculating an autocorrelation function from a detection signal (I(t) in FIG. 4) and processing the calculated autocorrelation function (G($\tau$) in FIG. 4). Specifically, according to the Wiener-Khintchine theorem, an autocorrelation function is calculated and thereafter performing Fourier transform of the autocorrelation function, whereby a power spectrum of I(t) is acquired. Note that, with the second processing method using the autocorrelation function, even when the detected detection signal is a signal in the present disclosure not having periodicity, it is possible to accurately obtain a power spectrum. It is possible to acquire desired blood flow information by processing the acquired power spectrum.

More specifically, in the second processing method, calculation of an autocorrelation function is performed on the detection signal in each predetermined time range to acquire a plurality of autocorrelation functions. Further, in the second processing method, according to the Wiener-Khintchine theorem, the FFT is performed on the calculated autocorrelation functions to acquire a plurality of power spectra (P(f) in FIG. 4), which are functions of frequencies. Since the power spectrum is proportional to presence density of a particle moving at speed corresponding to a frequency, by performing integration processing on the acquired power spectrum in a predetermined frequency range, it is possible to obtain relative density of the particle in blood vessels present in a predetermined speed range. Further, by acquiring relative densities of a plurality of particles from a plurality of power spectra in different time ranges, it is possible to acquire a waveform indicating a change with time of the relative densities of the particles. By performing interpolation processing or the like on the obtained waveform, it is possible to acquire a pulse waveform (not illustrated) indicating a change in the relative densities of the particles due to a pulse and calculate a pulse rate and the like as well from the pulse waveform. Note that, in the first processing method and the second processing method explained above, different methods are respectively used in a method of calculating a power spectrum and a method of calculating blood flow information after calculating the power spectrum. However, in the embodiment of the present disclosure, a combination of the methods is optional. That is, in the embodiment of the present disclosure, it is also possible to calculate a power spectrum with the method indicated by the first processing method and thereafter calculate relative density of a particle in the predetermined speed range with the second processing method. In the embodiment of the present disclosure, it is also possible to calculate a power spectrum with the method indicated by the second processing method and thereafter calculate an average blood flow rate indicated by the first processing method.

Note that, in embodiments of the present disclosure explained below, biological information is not limited to the blood flow information that can be acquired as explained above. In the embodiment of the present disclosure, the biological information may be blood alcohol concentration, a blood glucose level, or the like based on, for example, an infrared light absorption spectrum due to an alcohol component or sugar in blood of a person to be measured and is not particularly limited if the biological information is biological information relating to the body of the person to be measured.

<<2. First Embodiment>>

A first embodiment of the present disclosure explained below is an embodiment that makes it possible to easily acquire, with a biological information sensor that acquires blood flow information of a person to be measured and a camera that images the person to be measured, blood flow information of a specific part of the body of the person to be measured without attaching the biological information sensor. Note that, in the following explanation, it is assumed that blood flow information is acquired as biological information.

<2.1. Configuration of the information processing system 10 according to the first embodiment>

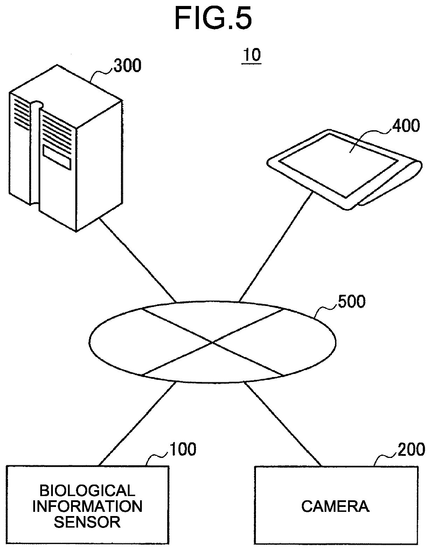

First, an example of the configuration of an information processing system according to the first embodiment of the present disclosure is explained with reference to FIG. 5. FIG. 5 is an explanatory diagram for explaining an example of the information processing system 10 according to this embodiment. As illustrated in FIG. 5, the information processing system 10 according to this embodiment includes the biological information sensor 100, the camera 200, the server 300, and a display device 400, which are communicably connected to one another via a network 500. Specifically, the biological information sensor 100, the camera 200, the server 300, and the display device 400 are connected to the network 500 via a not-illustrated base station or the like (for example, a base station of a cellular phone, an access point of a wireless LAN (Local Area Network), or the like). Note that, as a communication scheme used in the network 500, any scheme can be applied irrespective of wire or wireless (for example, WiFi (registered trademark), Bluetooth (registered trademark) and the like). However, it is desirable to use a communication scheme that can maintain stable operation. An overview of the devices included in the information processing system 10 according to this embodiment is explained below.

(Biological information sensor 100)

The biological information sensor 100 is a device that detects biological information such as blood flow information of a person to be measured and information concerning components in blood of the person to be measured such as blood alcohol concentration and a blood glucose level. In this embodiment, sensing data acquired by such a biological information sensor 100 is output to the server 300 explained below and processed. In this embodiment, the biological information sensor 100 is provided in noncontact with the body of the person to be measured and is provided in, for example, a wall in a room or the inside of a car where the person to be measured is present. Note that a detailed configuration of the biological information sensor 100 is explained below.

(Camera 200)

The camera 200 is a device that images the person to be measured using various members, for example, an imaging element (a light receiving element) such as a CCD (Charge Coupled Device) or a CMOS (Complementary Metal Oxide Semiconductor) that detects visible light and a lens for controlling focusing of an object image on the imaging element and generates a captured image of the person to be measured. Note that, in this embodiment, it is requested to associate an imaging range of the camera 200 and a detection range of the biological information sensor 100 in advance. Details of the association are explained below. Further, a detailed configuration of the camera 200 is explained below.

(Server 300)

The server 300 is configured by, for example, a computer. The server 300 is owned by, for example, a service provider for providing a service according to this embodiment. The server 300 can provide a service to users (including the person to be measured), that is, provide blood flow information (biological information) and the like. Specifically, the server 300 calculates, based on sensing data of the biological information sensor 100 and the camera 200, biological information such as blood flow information while linking the biological information with position information, which is information concerning an acquisition part of the blood flow information (for example, a part where light irradiated by the irradiating unit 102 of the biological information sensor 100 is reflected). Further, the server 300 provides the calculated blood flow information or the like to a user via the display device 400. Note that a detailed configuration of the server 300 is explained below.

(Display device 400)

The display device 400 is a device set in the vicinity of the user (including the person to be measured) to display the blood flow information (the biological information) or the like obtained by the server 300 to the user. For example, the display device 400 can be, for example, a liquid crystal display, an organic EL display (Organic Electro-Luminescence Display), or an HMD (Head Mounted Display). Further, the display device 400 may include an operation unit (not illustrated) that receives operation from the user and a speaker (not illustrated) that performs a sound output to the user. For example, the display device 400 can be a mobile terminal or the like such as a tablet PC (Personal Computer), a smartphone, a cellular phone, a laptop PC, or a notebook PC.
Note that, in FIG. 5, the information processing system 10 according to this embodiment is illustrated as including one biological information sensor 100, one camera 200, and one display device 400. However, in this embodiment, the information processing system 10 is not limited to this. For example, the information processing system 10 according to this embodiment may include a plurality of biological information sensors 100, a plurality of cameras 200, and a plurality of display devices 400. Further, the information processing system 10 according to this embodiment may include, for example, another communication device such as a relay device in transmitting information from the biological information sensor 100 to the server 300.

<2.2. Configuration of the biological information sensor 100 according to the first embodiment>

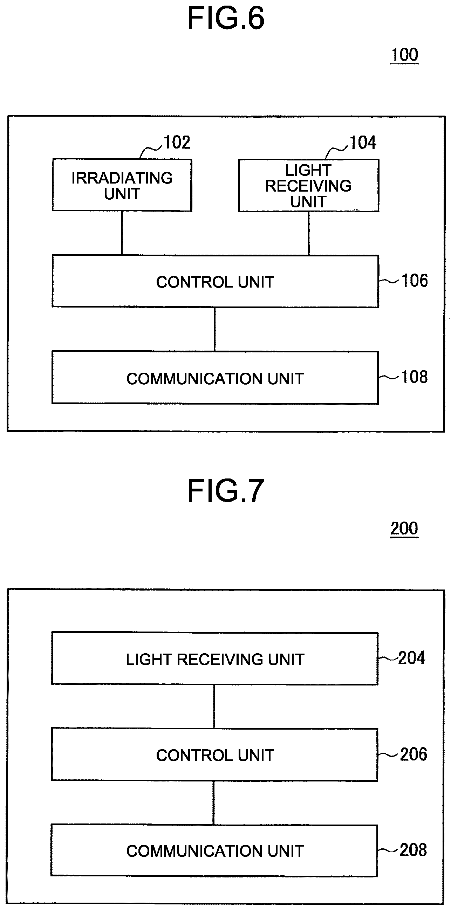

An example of the configuration of the biological information sensor 100 according to this embodiment is explained with reference to FIG. 6. FIG. 6 is a block diagram illustrating an example of the configuration of the biological information sensor 100 according to this embodiment. The biological information sensor 100 according to this embodiment is a device that performs measurement of blood flow information (biological information) on the person to be measured. Specifically, the biological information sensor 100 mainly includes, as illustrated in FIG. 6, the irradiating unit 102, the light receiving unit 104, a control unit 106, and a communication unit 108. The functional units included in the biological information sensor 100 are explained below.

(Irradiating unit 102)

The irradiating unit 102 irradiates the measurement region (a part of the body) 700 of the person to be measured with irradiation light having a predetermined wavelength. A wavelength of the irradiation light irradiated by the irradiating unit 102 can be selected as appropriate. The irradiating unit 102 irradiates, for example, light having a wavelength of approximately 800 nm to 900 nm. As the irradiating unit 102, a laser device or the like can be used in order to irradiate coherent light. Timing, an irradiation time, an irradiation interval, intensity, and the like for the irradiating unit 102 to irradiate the irradiation light can be controlled by the control unit 106 explained below. Note that, as explained above, the irradiating unit 102 is provided in noncontact with the person to be measured. Further, in this embodiment, the irradiating unit 102 preferably irradiates, with light, a region (a predetermined part) where blood vessels of the person to be measured are concentratedly present and preferably irradiates, for example, a cheek, a fingertip, or a palm with light.

(Light receiving unit 104)

The light receiving unit (a second light receiving unit) 104 detects light reflected from the measurement region 700 of the person to be measured. The light receiving unit 104 includes, for example, a photodiode (photo detector; PD), converts the intensity of the received light into an electric signal, and outputs the electric signal to the server 300 explained below. Note that, as the light receiving unit 104, a CCD (Charge Coupled Device) sensor, a CMOS (Complementary Metal Oxide Semiconductor) sensor, and the like can also be used. A plurality of elements (light receiving elements) such as the photodiode or the sensor explained above can be provided in the biological information sensor 100. Timing and the like for the light receiving unit 104 to output (read out) a detection signal are controlled by the control unit 106 explained below. Note that, as explained above, the light receiving unit 104 is provided in noncontact with the person to be measured.

(Control unit 106)

Based on a predetermined synchronization signal or the like, the control unit 106 controls measurement in general in the biological information sensor 100, for example, controls an irradiation pattern (irradiation timing, an irradiation time, and an irradiation interval) of the irradiating unit 102 and controls readout (sampling) timing of the light receiving unit 104. For example, the control unit 106 controls, according to the operation of the information processing system 10, an irradiation frequency of the irradiating unit 102 and a sampling frequency of the light receiving unit 104 synchronizing with the irradiation frequency. The control unit 106 may further include a not-illustrated storing unit. Various programs, parameters, and the like for controlling the irradiating unit 102 and the like may be stored in the storing unit. Further, in order to link a detection signal with time and output the detection signal to the server 300, the control unit 106 may incorporate a clock mechanism (not illustrated) for grasping accurate time. Specifically, the control unit 106 is realized by, for example, a CPU (Central Processing Unit), a ROM (Read Only Memory), and a RAM (Random Access Memory). Note that a part or all of functions performed by the control unit 106 may be performed in the server 300 explained below.

(Communication unit 108)

The communication unit 108 is provided in the biological information sensor 100 and can perform transmission and reception of information to and from an external device such as the server 300. In other words, the communication unit 108 can be considered a communication interface having a function of transmitting and receiving data. Note that the communication unit 108 is realized by a communication device such as a communication antenna or a transmission and reception circuit port.
Further, the biological information sensor 100 may include other functional units not illustrated in FIG. 6 besides the irradiating unit 102 and the like explained above.

<2.3. Configuration of the camera 200 according to the first embodiment>

An example of the configuration of the camera 200 according to this embodiment is explained with reference to FIG. 7. FIG. 7 is a block diagram illustrating the example of the configuration of the camera 200 according to this embodiment. The camera 200 according to this embodiment is a device that images the person to be measured as explained above. Specifically, the camera 200 mainly includes, as illustrated in FIG. 7, a light receiving unit 204, a control unit 206, and a communication unit 208. The functional units included in the camera 200 are explained below.

(Light receiving unit 204)

The light receiving unit (a first light receiving unit) 204 includes, for example, a plurality of PDs, CCD sensors, or CMOS sensors (light receiving elements) and acquires a captured image (a visible light image or the like) of the person to be measured. Timing and the like for the light receiving unit 104 to output (read out) a detection signal are controlled by the control unit 106 explained below. Note that, in this embodiment, the positions of the biological information sensor 100 and the camera 200 can be fixed in advance. Elements of the light receiving unit 104 of the biological information sensor 100 and elements of the light receiving unit 204 of the camera 200, that can receive light from the same part of the body of the person to be measured, are associated with each other. Consequently, biological information acquired from regions of the captured image by the camera 200 and regions of the body of the person to be measured can be linked.

(Control unit 206)

Based on a predetermined synchronization signal or the like, the control unit 206 controls measurement in general in the camera 200, for example, controls readout (sampling) timing of the light receiving unit 204. Specifically, the control unit 206 is realized by, for example, a CPU, a ROM, and a RAM.

(Communication unit 208)

The communication unit 208 is provided in the camera 200 and can perform transmission and reception of information to and from an external device such as the server 300. In other words, the communication unit 208 can be considered a communication interface having a function of performing transmission and reception of data. Note that the communication unit 208 is realized by a communication device such as a communication antenna, a transmission and reception circuit, or a port.
Further, the camera 200 may include other functional units not illustrated in FIG. 7 besides the light receiving unit 204 and the like explained above.

<2.4. Configuration of the server 300 according to the first embodiment>

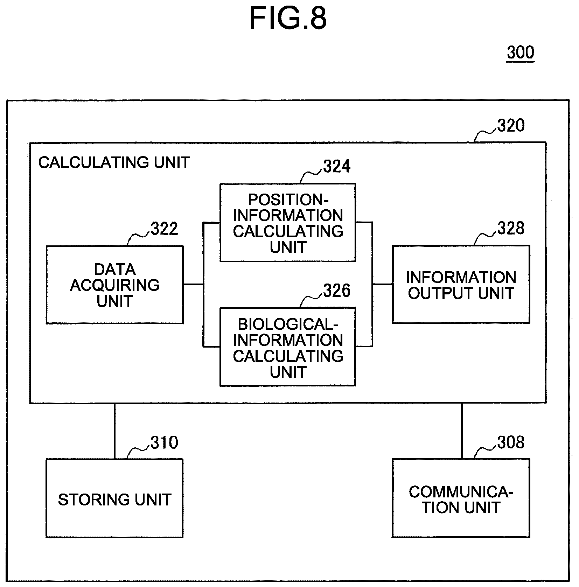

The configuration of the server 300 according to this embodiment is explained with reference to FIG. 8. FIG. 8 is a block diagram illustrating an example of the configuration of the server 300 according to this embodiment. The server 300 is a device that can acquire blood flow information (biological information), position information, and the like using sensing data obtained by the biological information sensor 100 or the like. The server 300 mainly includes, as illustrated in FIG. 8, a communication unit 308, a storing unit 310, and a calculating unit 320. The functional units included in the server 300 are explained below.

(Communication unit 308)

The communication unit 308 is provided in the server 300 and can perform transmission and reception of information to and from an external device such as the biological information sensor 100. In other words, the communication unit 308 can be considered a communication interface having a function of performing transmission and reception of data. Note that the communication unit 308 is realized by a communication device such as a communication antenna, a transmission and reception circuit, or a port.

(Storing unit 310)

In the storing unit 310, a program and various data used in processing in the calculating unit 320 explained below and the blood flow information (the biological information), the position information, and the like acquired by the calculating unit 320 are stored. In the storing unit 310, besides these data and the like, various parameters, an interim of processing, and the like that need to be saved in performing some processing may be stored as appropriate. Further, the calculating unit 320 and the like can freely access the storing unit 310 and write data in and read out data from the storing unit 310.

(Calculating unit 320)

The calculating unit 320 processes sensing data obtained by the biological information sensor 100 or the like to thereby acquire the blood flow information (the biological information), the position information, and the like. The acquired blood flow information can be output to the storing unit 310 or can be output to the display device 400. Specifically, as illustrated in FIG. 8, the calculating unit 320 mainly includes a data acquiring unit 322, a position-information calculating unit 324, a biological-information calculating unit 326, and an information output unit 328. The functional units included in the calculating unit 320 are explained below.

Data acquiring unit 322

The data acquiring unit 322 acquires sensing data transmitted from the biological information sensor 100 and the camera 200 and outputs the acquired sensing data to the position-information calculating unit 324 and the biological-information calculating unit 326 explained below.

Position-information calculating unit 324

The position-information calculating unit 324 calculates position information based on a captured image of the person to be measured obtained by processing the sensing data acquired from the camera 200. For example, the position-information calculating unit 324 can estimate, by matching a known human body shape model and the captured image, which region of the human body a region included in the captured image is, that is, position information. Position information corresponding to regions of the captured image by the camera 200 is output to the biological-information calculating unit 326 while being linked with information concerning the elements of the light receiving unit 204 of the camera 200 corresponding to the regions of the captured image.

Biological-information calculating unit 326

The biological-information calculating unit 326 can calculate, as biological information, blood flow information based on an interference light component included in the sensing data acquired from the biological information sensor 100 or blood alcohol concentration or a blood glucose level based on an absorption spectrum obtained from the sensing data. For example, the biological-information calculating unit 326 calculates, using the sensing data acquired from the biological information sensor 100, blood flow information (for example, a blood flow rate and particle density in blood vessels present in a predetermined speed range). As a method of the calculation, for example, the first processing method and the second processing method explained above can be used. The blood alcohol concentration or the blood glucose level can be calculated by the biological-information calculating unit 326 based on absorption of light having a predetermined wavelength by organic matters such as alcohol and sugar present in blood of the person to be measured. The blood flow information and the like calculated by the biological-information calculating unit 326 are linked with the position information via information concerning the elements of the light receiving unit 104 of the biological information sensor 100 that receives the sensing data, on which the blood flow information is based, and information concerning the elements of the light receiving unit 204 of the camera 200 corresponding to the elements. Further, the linked blood flow information is output to the information output unit 328 and the storing unit 310 explained below.

In this embodiment, by using the position information linked with the blood flow information and the like, it is possible to continuously extract blood flow information and the like in a predetermined position set in advance. As a result, it is possible to acquire a change with time of the blood flow information and the like in the predetermined position. Therefore, the biological-information calculating unit 326 may perform interpolation processing or the like on a waveform of the change with time of the blood flow information explained above to thereby calculate a pulse waveform and calculate a pulse rate from the pulse waveform. Further, the biological-information calculating unit 326 may estimate a state of the body of the person to be measured based on the change with time of the blood flow information and the like. In addition, the biological-information calculating unit 326 may calculate, besides the pulse rate, a consumed calorie, a blood pressure, a vascular age, a respiration rate, an HRV (Heart Rate Variability) indicator, and the like based on the change with time of the blood flow information and the like.

The HRV indicator is briefly explained. As explained above, in this embodiment, it is possible to acquire a change with time of blood flow information and can further acquire a pulse signal from such a change with time of the blood flow information. Peak intervals of a plurality of peaks appearing in the pulse wave signal are referred to as peak to peak intervals (PPIs). It is known that values of the PPIs fluctuate with time but, when a state of the person to be measured is stable, the values are substantially normally distributed. Therefore, by processing time series data of the PPIs, that is, a data group of the PPI values, it is possible to calculate various HRV indicators, which are indicators of a body state of the person to be measured.

Examples of the HRV indicator include an RMSSD (Root Mean Square Successive Difference), an SDNN (Standard Deviation of the Normal to Normal Interval), and an LF/HF. For example, the RMSSD is a square root of an average value of a square of a difference between PPI values adjacent to each other on a time sequence. The RMSSD is considered to be an indicator indicating a tension state of a vagus nerve, which is one of cranial nerves. For example, the SDNN is a standard deviation of a data group of PPI values in a predetermined period (for example, 120 seconds). The SDNN is considered to be an indicator indicating an activity state of an autonomic nerve system including both of a sympathetic nerve and a parasympathetic nerve. For example, the LF/HF is a ratio of a power spectrum of a low-frequency component (for example, 0.004 to 0.15 Hz) with respect to a power spectrum of a high-frequency component (for example, 0.15 to 0.4 Hz) of time series data of PPIs. The LF/HF is considered to be an indicator indicating a balance between the sympathetic nerve and the parasympathetic nerve. A high LF/HF indicates a state in which the sympathetic nerve is predominant. A low LF/HF indicates a state in which the parasympathetic nerve is predominant. Therefore, by being further processed, the HRV indicator can be used for evaluation of a sleep state, evaluation of a psychological stress degree, evaluation of a relax degree, evaluation of a concentration degree, and the like of the person to be measured.

Information output unit 328

The information output unit 328 controls the communication unit 308 to transmit, to the display device 400 and the like, the blood flow information (the biological information) and the like output from the position-information calculating unit 324 and the biological-information calculating unit 326 explained above.

Further, the server 300 may include an input unit (not illustrated) that receives operation from the user (including the person to be measured) who uses the information processing system 10 according to this embodiment.

The server 300 may be a device integrated with the biological information sensor 100 and the like explained above or may be a device separate from the biological information sensor 100 and the like explained above. In the latter case, the server 300 may be an information processing device such as a smartphone, a tablet, or a personal computer (PC) or may be an information processing device connected to another device (for example, a medical device). Further, the server 300 may be an information processing device set in, for example, a place apart from the person to be measured.

<2.5. Information processing method according to the first embodiment>

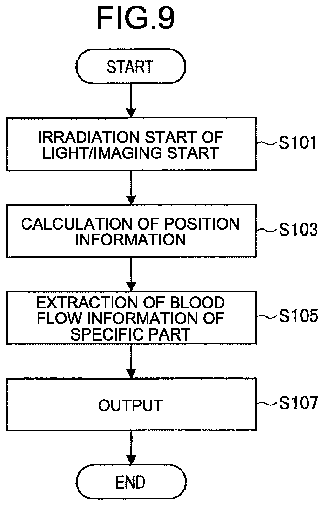

The details of the information processing system 10 according to the embodiment of the present disclosure and the devices included in the information processing system 10 are explained above. An information processing method according to this embodiment is explained with reference to FIG. 9. FIG. 9 is a flowchart illustrating an example of the information processing method according to this embodiment.

As illustrated in FIG. 9, the information processing method according to this embodiment includes a plurality of steps S101 to S107. Details of the steps included in the information processing method according to this embodiment are explained below.

(Step S101)

The information processing system 10 starts irradiation of the person to be measured with light and imaging, measures reflected light for acquiring blood flow information, and acquires a captured image of the person to be measured.

(Step S103)

The information processing system 10 matches the captured image of the person to be measured acquired in step S101 explained above with a known human body shape model to thereby calculate position information indicating which region of the human body a region included in the captured image is.

(Step S105)

The information processing system 10 extracts blood flow information of a predetermined specific part (for example, a cheek of the person to be measured). Specifically, as explained above, the information processing system 10 processes the reflected light (sensing data) acquired in step S101 explained above and calculates blood flow information. The information processing system 10 links the blood flow information with the position information via the information concerning the elements of the light receiving unit 104 of the biological information sensor 100 that receives the sensing data, on which the blood flow information is based, and the information concerning the elements of the light receiving unit 204 of the camera 200 corresponding to the elements. Further, the information processing system 10 extracts the blood flow information of the predetermined specific part by referring to position information linked with respective kind of blood flow information.

(Step S107)

The information processing system 10 outputs the blood flow information of the predetermined specific part extracted in step S105 explained above to the user.

As explained above, according to this embodiment, it is possible to easily acquire the blood flow information of the specific part of the body of the person to be measured without attaching the biological information sensor 100. As a result, according to this embodiment, it is possible to avoid hindrance to a motion of the person to be measured. Further, in this embodiment, from a specific part where measurement can be performed in a suitable state, it is possible to continuously or intermittently acquire blood flow information, that is, acquire a change with time of blood flow information of the specific part. As a result, according to this embodiment, it is possible to further improve accuracy of state information concerning a state of the body of the person to be measured obtained by analyzing the change with time of the blood flow information.

<<3. Second Embodiment>>

Incidentally, in the first embodiment explained above, the blood flow information and the captured image for calculating the position information are acquired using the different devices (sensors). Therefore, in the first embodiment explained above, in order to link the position information and the blood flow information, it is requested to associate the imaging range of the camera 200 and the detection range of the biological information sensor 100. However, it is troublesome to perform such association. Since a positional relation between the biological information sensor 100 and the camera 200 is fixed, the position of the person to be measured is also limited. Further, in the first embodiment, since the information processing system 10 includes the biological information sensor 100 and the camera 200, it is difficult to avoid an increase in power consumption of the information processing system 10 and complication of the information processing system 10.

Therefore, in the second embodiment of the present disclosure explained below, a biological information sensor 100a is caused to function as a TOF (Time Of Flight) sensor and a sensor that acquires blood flow information. The TOF sensor can acquire depth information of an object (a person to be measured) by, for example, irradiating the object with irradiation light having a predetermined cycle, detecting the light (reflected light) reflected on the object, and detecting a phase difference and a time difference between the irradiation light and the reflected light. The depth information of the object is information concerning depth, which is one of kinds of information concerning distances from the TOF sensor to points on an object surface. By aggregating the depth information of a plurality of points on the object surface, it is possible to treat the depth information as shape information concerning an uneven shape of the object surface, that is, information concerning the external shape of the object.

Incidentally, when two sensors, that is, the TOF sensor and the sensor that acquires blood flow information are used, it is requested to perform the troublesome association explained above. Further, in this case, since the information processing system 10 includes the two sensors, it is difficult to avoid an increase in power consumption of the information processing system 10 and complication of the information processing system 10. In addition, in such a case, when wavelengths of lights used in the respective sensors are close, the lights interfere with each other and greatly affect a measurement result. Therefore, it is difficult to accurately acquire the depth information and the blood flow information.

However, in the second embodiment, it is possible to avoid performing the troublesome association by sharing an irradiating unit and a light receiving unit of the TOF sensor and the sensor that acquires blood flow information, that is, causing the biological information sensor 100a to function as the TOF sensor and the sensor that acquires blood flow information. Further, according to this embodiment, since the TOF sensor and the sensor that acquires blood flow information can be formed as one sensor, it is possible to avoid an increase in power consumption of an information processing system 10a and complication of the information processing system 10a. Further, according to this embodiment, since the two sensors are not used, mutual interference of lights does not occur. As a result, it is possible to accurately acquire the depth information and the blood flow information. Details of the second embodiment of the present disclosure are explained below.

<3.1. Configuration of the information processing system 10a according to the second embodiment>

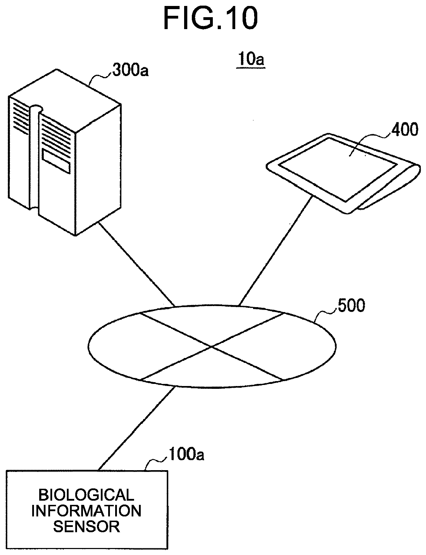

First, an example of the configuration of the information processing system 10a according to the second embodiment of the present disclosure is explained with reference to FIG. 10. FIG. 10 is an explanatory diagram for explaining an example of the information processing system 10a according to this embodiment. As illustrated in FIG. 10, the information processing system 10a according to this embodiment includes the biological information sensor 100a, a server 300a, and the display device 400, which are communicably connected to one another via the network 500. That is, the information processing system 10a according to this embodiment may not include the camera 200 unlike the first embodiment explained above. Note that, since the display device 400 is common to the first embodiment, explanation about the display device 400 is omitted. Only the biological information sensor 100a and the server 300a different from those in the first embodiment are explained.

(Biological information sensor 100a)

As in the first embodiment, the biological information sensor 100a is a device that detects biological information such as blood flow information, blood alcohol concentration, and a blood glucose level of the person to be measured. Further, in this embodiment, it is possible to calculate position information by processing sensing data acquired from the biological information sensor 100a. In this embodiment as well, the biological information sensor 100a is provided in noncontact with the body of the person to be measured. Note that a detailed configuration of the biological information sensor 100a is explained below.

(Server 300a)

As in the first embodiment, the server 300a is configured by, for example, a computer and can provide a service to users (including the person to be measured), that is, provide blood flow information (biological information). Note that a detailed configuration of the server 300a is explained below.

Note that, in this embodiment as well, the information processing system 10a may include a plurality of biological information sensors 100a and a plurality of display devices 400

<3.2. Configuration of the biological information sensor 100a according to the second embodiment>

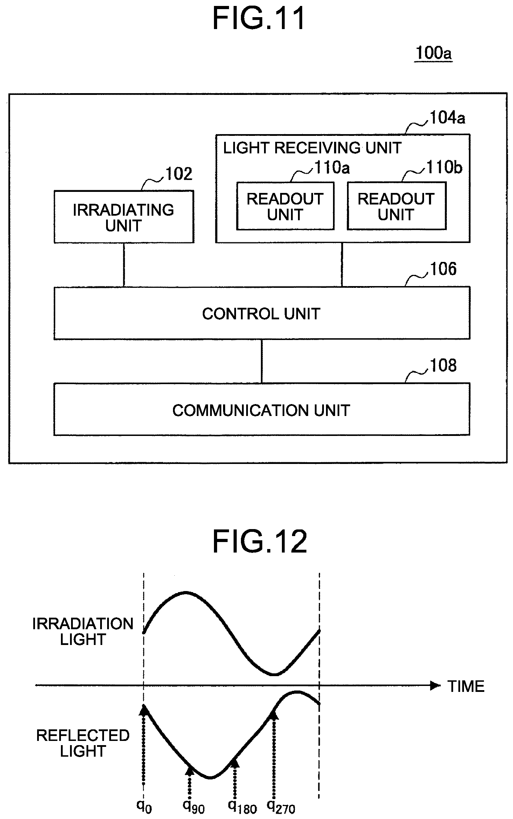

An example of the configuration of the biological information sensor 100a according to this embodiment is explained with reference to FIG. 11. FIG. 11 is a block diagram illustrating an example of the configuration of the biological information sensor 100a according to this embodiment. Specifically, the biological information sensor 100a mainly includes, as illustrated in FIG. 11, the irradiating unit 102, a light receiving unit 104a, the control unit 106, and the communication unit 108. Note that, since the irradiating unit 102, the control unit 106, and the communication unit 108 are common to the first embodiment, explanation about the irradiating unit 102, the control unit 106, and the communication unit 108 is omitted. Only the light receiving unit 104a different from that in the first embodiment is explained.

(Light receiving unit 104a)

As in the first embodiment, the light receiving unit 104a detects light reflected from the measurement region 700 of the person to be measured. The light receiving unit 104a includes elements (light receiving elements) such as a plurality of photodiodes or sensors provided in a matrix shape. The elements include readout units (first and second readout units) 110a and 110b, timing of which for outputting (reading out) a detection signal is controlled by the control unit 106. Specifically, the readout units 110a and 110b can read out, at different timings from each other, lights (specifically, charges) detected by the elements.

A principle of a method of calculating depth information by a phase difference using the biological information sensor 100a is explained with reference to FIG. 12. FIG. 12 is an explanatory diagram for explaining a principle of a method of calculating depth information. Specifically, FIG. 12 schematically illustrates temporal fluctuations of intensities of irradiation light and reflected light in the biological information sensor 100a.

As illustrated in FIG. 12, the biological information sensor 100a irradiates, from the irradiating unit 102, the person to be measured with light, the intensity of which is modulated to cyclically fluctuate. The irradiated light is reflected in a part of the body of the person to be measured and detected by the light receiving unit 104a of the biological information sensor 100a as reflected light. As illustrated in FIG. 12, the detected reflected light (a lower stage of FIG. 12) has a phase difference with respect to the irradiation light (an upper stage of FIG. 12). The phase difference increases if the distance from the biological information sensor 100a to the person to be measured is large and decreases if the distance from the biological information sensor 100a to the person to be measured is small.

Therefore, for example, the biological information sensor 100a senses light intensities in four phases (0 degree, 90 degrees, 180 degrees, and 270 degrees) of the reflected light. It is possible to calculate a phase difference (phase) by inputting sensing data ($q_0$, $q_{90}$, $q_{180}$, $q_{270}$) to the following Expression (1). Further, by using the phase difference calculated in this way and a wavelength (range) of light, according to the following Expression (1), it is possible to obtain depth information (distance) (distance information) to the person to be measured.

$$I = q_0 - q_{180}$$
$$Q = q_{90} - q_{270}$$
$$phase = tan^{-1}\left(\frac{Q}{I}\right)$$
$$distance = \frac{phase \times range}{2\pi}$$

$$\ldots(1)$$

Note that the depth information can be acquired for each element of the light receiving unit 104a. Therefore, the position of the relevant element and the depth information can be linked one to one.

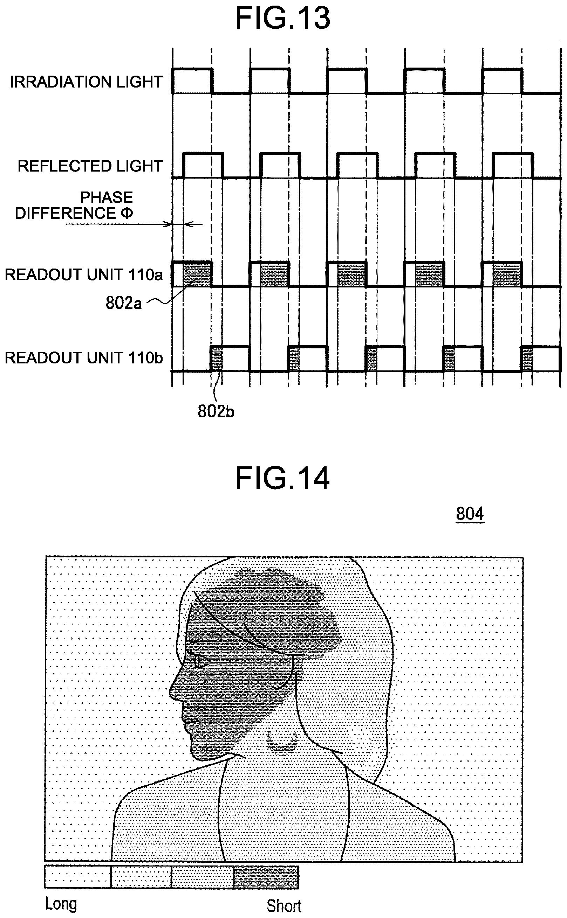

Further, in the light receiving unit 104a according to this embodiment, as explained above, the elements include the readout units 110a and 110b that differentially operate each other and read out lights. Specifically, the readout units 110a and 110b according to this embodiment operate in periods having the same length but operate to have a phase difference of 180 degrees each other (see FIG. 13).

A method of calculating depth information according to this embodiment is explained with reference to FIG. 13 and FIG. 14. FIG. 13 is an explanatory diagram for explaining a method of calculating depth information using the biological information sensor 100a according to this embodiment. FIG. 14 is an explanatory diagram for explaining an example of an image 804 based on depth information according to this embodiment.

In FIG. 13, irradiation light (a first stage of FIG. 13) and reflected light (a second stage of FIG. 13) are illustrated as pulse-like lights to be clearly seen. A phase difference between the irradiation light and the reflected light is $\phi$. Further, in FIG. 13, the operations of the readout unit 110a (a third stage of FIG. 13) and the readout unit 110b (a fourth stage of FIG. 13) are illustrated. The readout units 110a and 110b operate in periods having convex on the upper side. Therefore, as illustrated in FIG. 13, the periods in which the readout units 110a and 110b respectively operate do not overlap. Therefore, it is seen that the readout units 110a and 110b differentially operate each other (that is, operate at different timings).

As illustrated in FIG. 13, when the reflected light has the phase difference $\phi$ with respect to the irradiation light, the readout units 110a and 110b can detect the reflected light in regions 802a and 802b indicated by gray in FIG. 13. Specifically, by respectively integrating the intensities of the lights detected by the readout units 110a and 110b, it is possible to obtain a light reception signal equivalent to the area of the region 802a and the region 802b in FIG. 13. As it is evident from FIG. 13, a difference between an integration value in the readout unit 110a and an integration value in the readout unit 110b changes according to the phase difference $\phi$ of the reflected light. Therefore, in this embodiment, it is possible to calculate the difference between the integration values of the readout units 110a and 110b, calculate the phase difference $\phi$ based on the calculated difference, and further calculate depth information. Note that, in this embodiment, it is also possible to calculate the phase difference $\Phi$ using a ratio of the integration value rather than the difference between the integration values and calculate depth information.

With the depth information obtained in this way, it is possible to acquire the image 804 based on depth information of the person to be measured (a face of a person) illustrated in FIG. 14. The image 804 based on the depth information is an image obtained by projecting, on a plane, information obtained by linking depth information obtained based on reflected lights reflected at points on the surface of the body of the person to be measured with the positions of the elements of the light receiving unit 104a. Therefore, the image 804 can be considered an image illustrating an uneven shape of the external shape of the person to be measured.

Note that, in this embodiment, the depth information of the person to be measured is not limited to be calculated according to the phase difference explained above and may be calculated according to a time difference from the irradiation of the light by the irradiating unit 102 until the reception of the light by the light receiving unit 104a.

<3.3. Configuration of the server 300a according to the second embodiment>

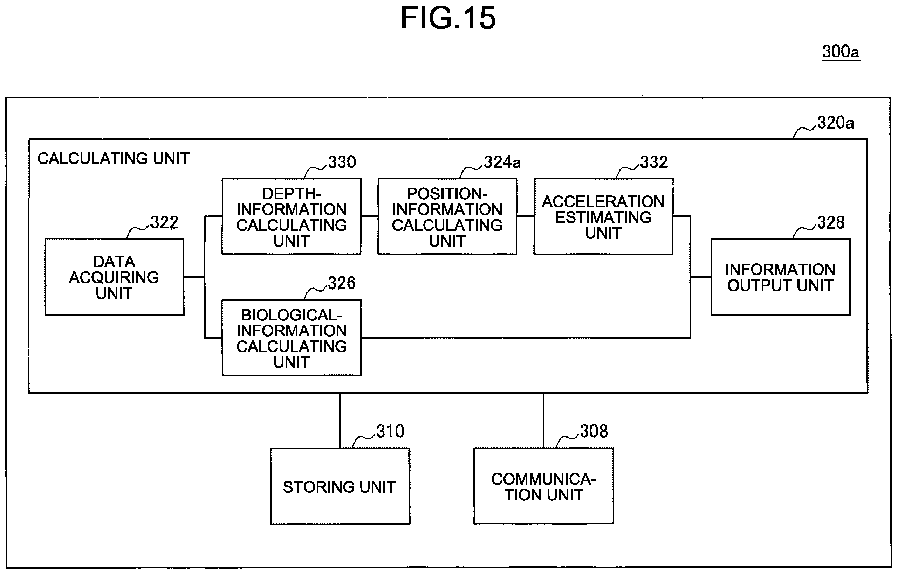

The configuration of the server 300a according to this embodiment is explained with reference to FIG. 15. FIG. 15 is a block diagram illustrating an example of the configuration of the server 300a according to this embodiment. The server 300a mainly includes, as illustrated in FIG. 15, the communication unit 308, the storing unit 310, and a calculating unit 320a. Note that the communication unit 308 and the storing unit 310 are common to the first embodiment. Therefore, explanation about the communication unit 308 and the storing unit 310 is omitted. Only the calculating unit 320a different from that in the first embodiment is explained.

(Calculating unit 320a)

The calculating unit 320a processes sensing data obtained by the biological information sensor 100a or the like to thereby acquire blood flow information and position information. Specifically, as illustrated in FIG. 15, the calculating unit 320a mainly includes the data acquiring unit 322, a position-information calculating unit 324a, the biological-information calculating unit 326, the information output unit 328, a depth-information calculating unit 330, and an acceleration estimating unit 332. Note that the data acquiring unit 322, the biological-information calculating unit 326, and the information output unit 328 are common to the first embodiment. Therefore, explanation about the data acquiring unit 322, the biological-information calculating unit 326, and the information output unit 328 is omitted. Only the position-information calculating unit 324a, the depth-information calculating unit 330, and the acceleration estimating unit 332 different from those in the first embodiment are explained.

Depth-information calculating unit 330

As explained above, the depth-information calculating unit 330 calculates a phase difference, which is a difference between a phase of the light irradiated by the irradiating unit 102 and a phase of the light received by the light receiving unit 104a to thereby calculate depth information of the person to be measured. Alternatively, the depth-information calculating unit 330 calculates a time difference from the irradiation of the light by the irradiating unit 102 until the light reception of the light by the light receiving unit 104a to thereby calculate depth information of the person to be measured. Note that, in the former case, as explained above, the depth-information calculating unit 330 can calculate the phase difference based on the intensities of the lights read out by the readout units 110a and 110b of the light receiving unit 104a.

Position-information calculating unit 324a

The position-information calculating unit 324a calculates position information based on the image 804 illustrating the external shape of the person to be measured based on a plurality of kinds of depth information obtained from the depth-information calculating unit 330. For example, the position-information calculating unit 324a can estimate, by matching a known human shape model and the image 804, which region of a human a region of the image 804 is. Position information corresponding to regions of the image 804 is output to the biological-information calculating unit 326 while being linked with information concerning the elements of the light receiving unit 104a of the biological information sensor 100a corresponding to the regions of the image 804. Blood flow information (biological information) calculated by the biological-information calculating unit 326 is linked with the position information via the information concerning the elements.

Acceleration estimating unit 332

The acceleration estimating unit 332 estimates motion acceleration of the person to be measured based on a change with time of depth information, that is, a change with time of the external shape of the person to be measured. Note that the acceleration estimating unit 332 may not be provided in the calculating unit 320a. Details of processing of the acceleration estimating unit 332 are explained below.

<3.4. Information processing method according to the second embodiment>

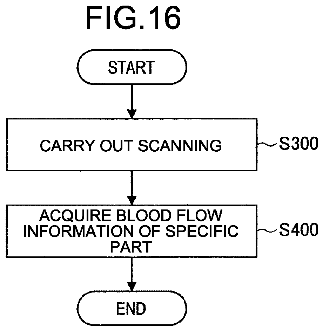

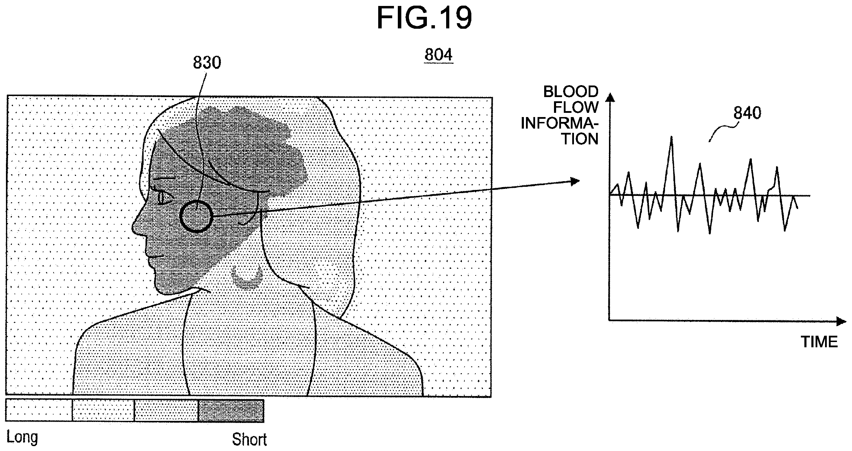

The details of the information processing system 10a according to the second embodiment of the present disclosure and the devices included in the information processing system 10a are explained above. An information processing method according to this embodiment is explained with reference to FIG. 16 to FIG. 19. FIG. 16 to FIG. 18 are flowcharts illustrating an example of the information processing method according to this embodiment. FIG. 19 is an explanatory diagram for explaining an example of the image 804 and the detection signal 800 based on depth information according to this embodiment.

As illustrated in FIG. 16, the information processing method according to this embodiment roughly includes step S300 and step S400. Details of the steps included in the information processing method according to this embodiment are explained below.

(Step S300)

The information processing system 10a carries out scanning (irradiation of light/reception of light) on the body of the person to be measured.

(Step S400)

The information processing system 10a acquires blood flow information of a specific part based on sensing data acquired in step S300.

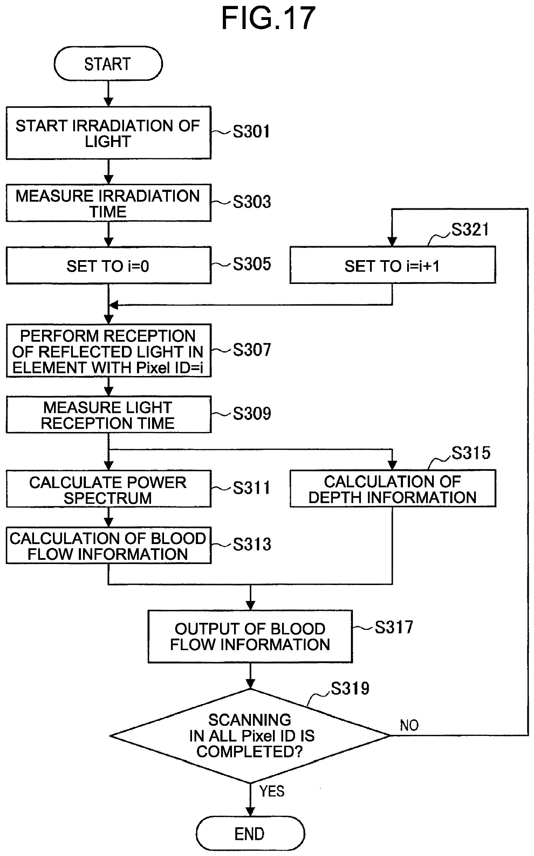

Further, details of step S300 and step S400 in FIG. 16 are explained. First, details of step S300 in FIG. 16 are explained. As illustrated in FIG. 17, step S300 of the information processing method according to this embodiment includes, for example, a plurality of steps S301 to S321. Details of the steps included in step S300 are explained below.

(step S301)

The information processing system 10a starts irradiation of the person to be measured with light.

(Step S303)

The information processing system 10a measures irradiation time.

(Step S305)

The information processing system 10a sets an element number (Pixel ID) of the light receiving unit 104 of the biological information sensor 100a to i=0.

(Step S307)

The information processing system 10a performs light reception of reflected light from the body of the person to be measured in an element with the element number i.

(Step S309)

The information processing system 10a measures light reception time.

(Step S311)

The information processing system 10a calculates a power spectrum in the element number i based on the reflected light received in step S307 using, for example, the first processing method or the second processing method explained above.

(Step S313)

The information processing system 10a calculates blood flow information in the element number i based on the power spectrum calculated in step S311.

(Step S315)

The information processing system 10a calculates depth information of the person to be measured in the element number i based on, for example, a time difference between the irradiation time measured in step S303 and the light reception time measured in step S309. Note that, in this embodiment, the depth information is not limited to be calculated based on such a time difference and may be calculated based on a phase difference between the irradiation light and the reflected light as explained above.

(Step S317)

The information processing system 10a outputs the blood flow information calculated in step S313 and the depth information calculated in step S315 to the storing unit 310 while linking the blood flow information and the depth information with the element number i.

(Step S319)

The information processing system 10a determines whether the scanning explained above (specifically, the processing in step S301 to step S319) has been performed in all the elements of the light receiving unit 104 of the biological information sensor 100a. When the scanning has been performed in all the elements, the information processing system 10a ends the processing. When the scanning has not been performed in all the elements, the information processing system 10a proceeds to step S321.

(Step S321)

The information processing system 10a sets the element number (Pixel ID) of the light receiving unit 104 to i = i + 1. That is, the information processing system 10a increments the element number for performing the processing by one. The information processing system 10a returns to step S307.

As explained above, in step S300, the information processing system 10a repeats the plurality of steps explained above to thereby scan the body of the person to be measured and acquires the blood flow information and the depth information for each element.

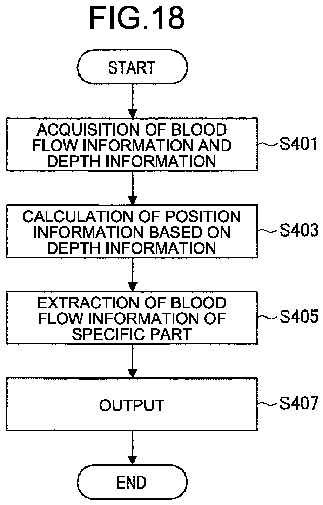

Details of step S400 in FIG. 16 are explained. As illustrated in FIG. 18, step S400 of the information processing method according to this embodiment includes, for example, the plurality of steps S401 to S407. Details of the steps included in step S400 are explained below.

(Step S401)

The information processing system 10a acquires, from the storing unit 310, the blood flow information and the depth information output in step S317 explained above.

(Step S403)

The information processing system 10 matches a plurality of kinds of depth information of the person to be measured (that is, information concerning the person to be measured) acquired in step S401 explained above or an image based on the plurality of kinds of depth information and a known human body shape model to thereby calculate position information indicating which region of the human body a target region of the depth information is. Specifically, the position information is calculated as position information on the human body to correspond to the depth information in the elements of the light receiving unit 104 of the biological information sensor 100a. Therefore, the position information is linked with element numbers (Pixel IDs) of the elements.

(Step S405)

The information processing system 10a extracts blood flow information of a predetermined specific part (for example, a cheek of the person to be measured). Specifically, the information processing system 10a extracts an element number of the specific part referring to the position information, which is linked with the depth information in the elements, calculated in step S403. Further, the information processing system 10a extracts blood flow information corresponding to the extracted element number out of the blood flow information acquired in step S403.

(Step S407)

The information processing system 10a outputs the blood flow information of the predetermined specific part extracted in step S405 explained above to the user.

Further, by repeating step S300 and step S400 explained above, it is possible to acquire a change with time of the blood flow information of the specific part of the body of the person to be measured. For example, it is possible to acquire a change with time 840 of blood flow information illustrated on the right side of FIG. 19 in a specific part 830 such as a cheek of the person to be measured in the image 804 based on a plurality of kinds of depth information of the person to be measured illustrated on the left side of FIG. 19. Note that, in this embodiment, the change with time 840 of the blood flow information is not limitedly acquired and, for example, a distribution of values relating to predetermined blood flow information on the body of the person to be measured (for example, a distribution of blood flow rates on the body of the person to be measured) may be acquired.

As explained above, according to this embodiment, it is possible to easily acquire the blood flow information of the specific part of the body of the person to be measured without attaching the biological information sensor 100. Further, according to this embodiment, since it is possible to acquire the change with time of the blood flow information of the specific part, it is possible to further improve accuracy of state information concerning a state of the body of the person to be measured obtained by analyzing the change with time of the blood flow information.

In addition, according to this embodiment, it is possible to avoid performing the troublesome association by causing the biological information sensor 100a to function as a TOF sensor and a sensor that acquires blood flow information. Further, according to this embodiment, since the TOF sensor and the sensor that acquires blood flow information can be formed as one sensor, it is possible to avoid an increase in power consumption of an information processing system 10a and complication of the information processing system 10a. Further, according to this embodiment, since the two sensors are not used, mutual interference of lights does not occur. As a result, it is possible to accurately acquire the depth information and the blood flow information.

In the embodiment explained above, by causing the biological information sensor 100a according to this embodiment to assume a function of the server 300a, the biological information sensor 100a may be used as a stand-alone device.

<3.5. Modification>

In the embodiment explained above, the biological information sensor 100 sometimes measures blood flow information of the person to be measured for a long time. Therefore, it is preferable that the power consumption of the biological information sensor 100 according to this embodiment is further reduced. Most of the power consumption in the biological information sensor 100 is consumed by the irradiating unit 102. Therefore, a modification in which the power consumption in the irradiating unit 102 can be reduced is explained.

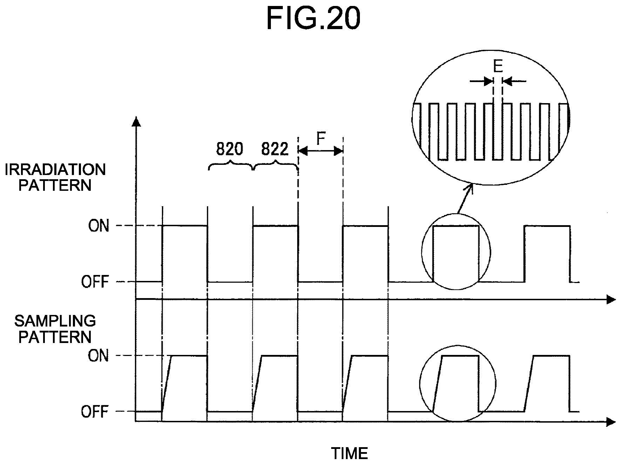

A measurement method according to this modification is specifically explained below with reference to FIG. 20. FIG. 20 is an explanatory diagram for explaining an example of an irradiation pattern and a sampling pattern according to a modification of this embodiment. Specifically, FIG. 20 schematically illustrates, from an upper stage to a lower stage, an irradiation pattern of the irradiating unit 102 and a readout (sampling) pattern of the light receiving unit 104 according to the modification. Note that, in FIG. 20, an enlarged diagram of a part of the irradiation pattern illustrated in the first stage is also illustrated.

In this modification, the irradiating unit 102 is controlled such that, as illustrated in the first stage of FIG. 20, the irradiation pattern of the irradiating unit 102 becomes a pattern in which a section 822 where the irradiating unit 102 regularly repeats irradiation and a section 820 where the irradiating unit 102 suspends the irradiation alternately appear. Consequently, it is possible to reduce the power consumption of the irradiating unit 102. Specifically, as illustrated in the first stage of FIG. 20, in the irradiation pattern of the irradiating unit 102, the section 822 where the irradiating unit 102 regularly repeats irradiation and the section 820 where the irradiating unit 102 suspends the irradiation alternately appear. In the section 822 where the irradiating unit 102 regularly repeats irradiation, as illustrated in an enlarged diagram of the section 822, the irradiating unit 102 intermittently irradiates the irradiation light at a first interval E. The section 822 is repeated at an interval F corresponding to the section 820 where the irradiation is suspended. The interval F is set to be longer than the interval E. Consequently, it is possible to reduce an irradiation period in which the irradiating unit 102 performs irradiation. Therefore, it is possible to reduce the power consumption in the irradiating unit 102.

As explained above, in this modification, the irradiation pattern of the irradiating unit 102 is formed as the pattern in which the section 822 where the irradiating unit 102 regularly repeats irradiation and the section 820 where the irradiating unit 102 suspends the irradiation alternately appear. Consequently, according to this modification, it is possible to reduce the power consumption in the irradiating unit 102.

<<4. Examples according to the embodiment of the present disclosure>>

The details of the information processing method in the second embodiment of the present disclosure are explained above. Examples of information processing according to an embodiment of the present disclosure are more specifically explained while explaining specific examples. Note that the examples explained below are only examples of the information processing according to the embodiment of the present disclosure. The information processing method according to the embodiment of the present disclosure is not limited to the examples explained below.

<4.1. Example 1>

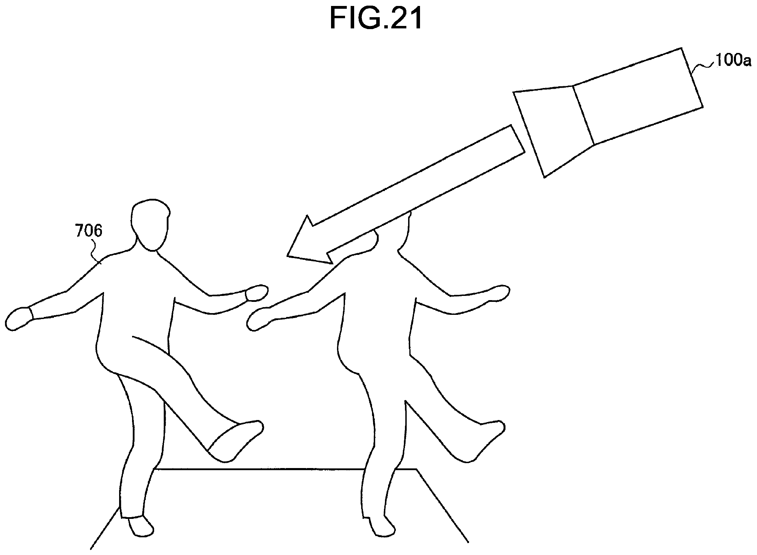

First, as an example 1, an example for estimating a consumed calorie of the person to be measured based on an action, a posture (a motion) and blood flow information of the person to be measured is explained with reference to FIG. 21. FIG. 21 is an explanatory diagram for explaining the example 1 according to the embodiment of the present disclosure.

In this example, as illustrated in FIG. 21, the biological information sensor 100a is provided on a wall in a room (for example, a gym) where a person to be measured 706 is present. In order to estimate a consumed calorie of the person to be measured 706 performing a motion, the biological information sensor 100a acquires sensing data for calculating blood flow information and position information (depth information).

In this example, it is possible to accurately estimate a consumed calorie of the person to be measured 706 by referring to not only the blood flow information but also acceleration relating to the motion of the person to be measured 706. Specifically, in this embodiment, acceleration relating to the motion of the person to be measured 706 is estimated by using the acceleration estimating unit 332 of the calculating unit 320a of the server 300. For example, the acceleration estimating unit 332 can estimate acceleration by the motion of the person to be measured 706 by analyzing depth information of the person to be measured 706, that is, a change with time of the external shape of the person to be measured 706. In this modification, motion acceleration of a specific part of the body of the person to be measured 706 may be estimated using the method explained above.

In this embodiment, since the blood flow information and the acceleration relating to the motion of the person to be measured 706 can also be referred to, it is possible to accurately estimate a consumed calorie of the person to be measured 706. Further, in this example, it is unnecessary to attach a sensor for acquiring sensing data relating to the blood flow information to the body of the person to be measured 706. Further, it is also unnecessary to attach a motion sensor for acquiring acceleration. Therefore, the motion of the person to be measured 706 is not hindered.

In this example, by referring to not only the consumed calorie but also position information and acceleration information and blood flow information corresponding to the position information, it is also possible to specify, for example, a part where a muscle is strained in the body of the person to be measured 706. Therefore, in this modification, since a muscle potential sensor or the like is not attached to a part of the body of the person to be measured 706, the motion of the person to be measured 706 is not hindered. It is possible to easily detect a state of the muscle of the person to be measured 706.

Note that the biological information sensor 100a is not limited to be set in a room such as a gym or a gymnastic hall and may be set in an athletic field or the outdoors where sports are performed. This modification can also be applied to a sports live broadcast (for example, in the sports live broadcast, detecting conditions of players and providing the conditions to viewers) and a sports analysis (for example, analyzing conditions of players belonging to an own team and other teams and making use of the conditions for instructions and tactics). Further, the biological information sensor 100a may be provided in a hall such as a concert hall or may be provided in a factory, an office, or the like.

<4.2. Example 2>

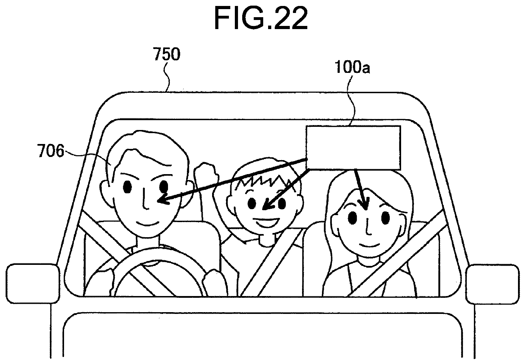

As an example 2, an example for detecting a sudden change in a blood flow volume due to a car sickness or the like of an occupant riding on an automobile is explained with reference to FIG. 22. FIG. 22 is an explanatory diagram for explaining an example 2 according to the embodiment of the present disclosure.

In this example, as illustrated in FIG. 22, the biological information sensor 100a is provided in a vehicle 750 on which the person to be measured 706 is riding. In order to estimate a state of a car sickness or the like of the riding person to be measured 706, the biological information sensor 100a acquires sensing data for calculating blood flow information and the like.

In this example, it is possible to acquire a change with time of blood flow information of a specific part suitable for acquiring the state of the car sickness of the person to be measured 706. Therefore, it is possible to improve accuracy of the estimation of the state of the motion sickness or the like of the person to be measured 706. Further, in this example, it is unnecessary to attach a sensor for acquiring sensing data relating to blood flow information to the body of the person to be measured 706. Therefore, it is possible to easily acquire the blood flow information without imposing a burden on the person to be measured 706.

Note that, in this example, it is possible to detect not only the car sickness but also a sudden change in a blood flow volume due to pallor of the face by anemia, heat attack, other diseases, and ill health. Further, information concerning driving for less easily causing the car sickness may be learned by linking the state of the car sickness of the occupant to which this example is applied and traveling data of the vehicle 750 acquired at that time and analyzing the state and the traveling data with artificial intelligence or the like. The information concerning the driving obtained by such learning can be utilized for vehicle control and automatic driving performed as a support for driving of a driver.

Further, this example is not limited to only providing the biological information sensor 100a in the vehicle 750 to thereby detect a sudden change in a blood flow volume due to a car sickness or the like of an occupant and can also be applied when the biological information sensor 100a is used together with a monitoring camera or a security camera (not illustrated) provided in a facility to detect a person in a tense state due to an attempt of a dishonest act to thereby detect a suspicious person. In addition, according to this example, since a psychological state (a relax degree or the like) of the person to be measured 706 can be detected, this example can also be applied to a commodity test or the like.

<4.3. Example 3>

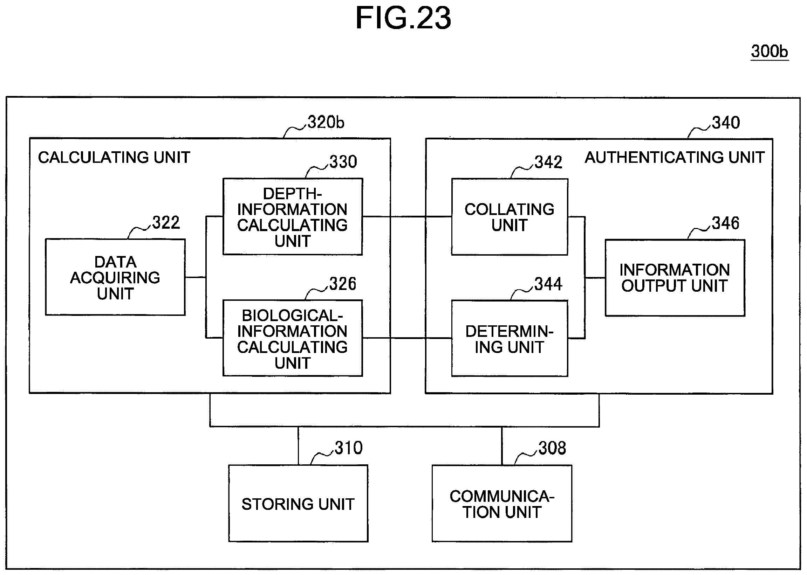

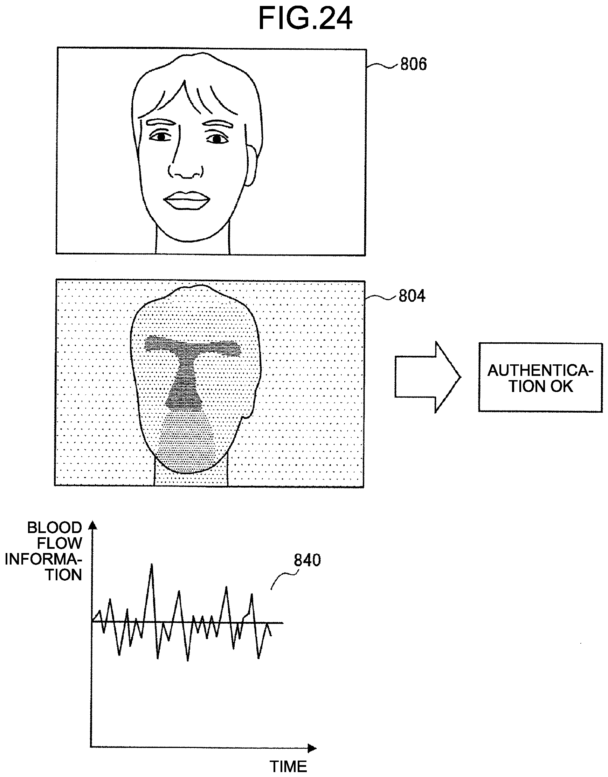

As an example 3, an example applied to face authentication is explained with reference to FIG. 23 and FIG. 24. FIG. 23 and FIG. 24 are explanatory diagrams for explaining an example 3 according to the embodiment of the present disclosure.

Incidentally, in recent years, in personal authentication using a face, that is, face authentication, authentication can be performed using not only a face image of an individual but also a plurality of kinds of depth information indicating a three-dimensional shape of the face of the individual. However, in such face authentication, a wrongdoer can break through the authentication by performing impersonation using a mask or the like simulating a three-dimensional shape of a face of a person to be authenticated.

Therefore, in this example, the breakthrough of the authentication by the impersonation is prevented by performing collation of a face using a plurality of kinds of depth information of a face of a person to be measured and determining, using the second embodiment of the present disclosure explained above, whether the face for which the depth information is used is an organism or a non-organism such as a mask (for example, whether a blood flow is successfully detected).

An example of a configuration according to this example is explained. In this example, the configuration of the information processing system 10 is the same as the configuration in the second embodiment explained above except that the server 300a is changed to a server 300b explained below. Therefore, only the configuration of the server 300b is explained.

The configuration of the server 300b according to this example is explained with reference to FIG. 23. The server 300b mainly includes, as illustrated in FIG. 23, the communication unit 308, the storing unit 310, a calculating unit 320b, and an authenticating unit 340. Note that the communication unit 308 and the storing unit 310 are common to the first and second embodiments. Therefore, explanation about the communication unit 308 and the storing unit 310 is omitted. Only the calculating unit 320b and the authenticating unit 340 different from those in the first and second embodiments are explained.

(Calculating unit 320b)

The calculating unit 320b acquires blood flow information, depth information, and the like by processing sensing data obtained by the biological information sensor 100a or the like. Specifically, as illustrated in FIG. 23, the calculating unit 320a mainly includes the data acquiring unit 322, the biological-information calculating unit 326, and the depth-information calculating unit 330. Note that, since these units are common to the second embodiment, explanation about the units is omitted.

(Authenticating unit 340)

The authenticating unit 340 can prevent a breakthrough of authentication by impersonation by matching a plurality of kinds of depth information of a face of a person to be measured or an image based on the plurality of kinds of depth information acquired from the calculating unit 320b and a three-dimensional shape of a face of a person acquired in advance to thereby perform person collation and by determining whether the face for which the depth information is used is an organism or a non-organism. Specifically, as illustrated in FIG. 23, the authenticating unit 340 mainly includes a collating unit 342, a determining unit 344, and an information output unit 346. The functional units included in the authenticating unit 340 are explained below.

Collating unit 342

The collating unit 342 matches the depth information acquired from the calculating unit 320b and the three-dimensional shape of the face of the person already acquired to collate the depth information and the three-dimensional shape and performs person collation. The collating unit 342 outputs a result of the collation to the information output unit 346. Note that, in this example, the collating unit 342 may perform the person collation using not only the depth information but also a captured image obtained by imaging the face of the person to be measured.

Determining unit 344

The determining unit 344 determines, based on the blood flow information acquired from the calculating unit 320b, whether the depth information is obtained from an organism. For example, when a change with time of the blood flow information is successfully detected, the determining unit 344 determines that the depth information is obtained from an organism. The determining unit 344 outputs a result of the determination to the information output unit 346 explained below.

Information output unit 346

The information output unit 346 performs authentication based on the results output from the collating unit 342 and the determining unit 344 explained above and controls the communication unit 308 to transmit a result of the authentication to the display device 400 and the like. That is, when the plurality of kinds of depth information (or the image based on the plurality of kinds of depth information) and the three-dimensional shape of the face of the person already acquired match and it is determined that the depth information is obtained from an organism because the change with time of the blood flow information is successfully detected, the authenticating unit 340 authenticates the relevant person.

In this example, for example, as illustrated in FIG. 24, the collation is performed on a captured image 806 of the face of the person to be measured and the image 804 based on the depth information and, when a change with time 840 of the blood flow information is successfully detected, the relevant person is collated.

As explained above, according to this example, it is possible to prevent the breakthrough of the authentication by impersonation by performing the collation of the face using the depth information of the face of the person to be measured and determining whether the face for which the depth information is used is an organism or a non-organism such as a mask.

In this example, it is possible to grasp blood flow information of which region on the body of the person to be measured is detected and it is possible to acquire an image (a speckle image illustrating a state of a blood flow) based on the blood flow information. Therefore, in this example, by grasping in advance shapes of an artery and a vein in a predetermined region such as the face of the person to be measured, the authentication may be performed by collating a speckle image of the predetermined region and the shapes grasped in advance. Further, in this example, by detecting a change with time of the speckle image, it is possible to determine whether the depth information is acquired from a non-organism and it is possible to prevent the breakthrough of the authentication by impersonation.

Note that the biological information sensor 100a according to this example may be provided in an entrance of a facility, a PC, a smartphone, a TV (television) apparatus, an AI (Artificial Intelligence) speaker, or the like.

<<5. Summary>>

As explained above, according to the embodiment of the present disclosure, it is possible to easily acquire blood flow information of a specific part of the body of a person to be measured without attaching the biological information sensor 100. As a result, according to this embodiment, it is possible to avoid hindrance to a motion of the person to be measured. Further, in this embodiment, from a specific part where measurement can be performed in a suitable state, it is possible to continuously or intermittently acquire blood flow information, that is, acquire a change with time of blood flow information of the specific part. As a result, according to this embodiment, it is possible to further improve accuracy of state information concerning a state of the body of the person to be measured obtained by analyzing the change with time of the blood flow information.

Note that the embodiment explained above is not limited to be used for diagnosis and authentication for a human and can also be applied to diagnosis and the like for an animal such as a pet. In this case, even for an animal to which it is difficult to attach a sensor, it is possible to easily acquire biological information from a specific part necessary for the diagnosis.

<<6. About a hardware configuration>>

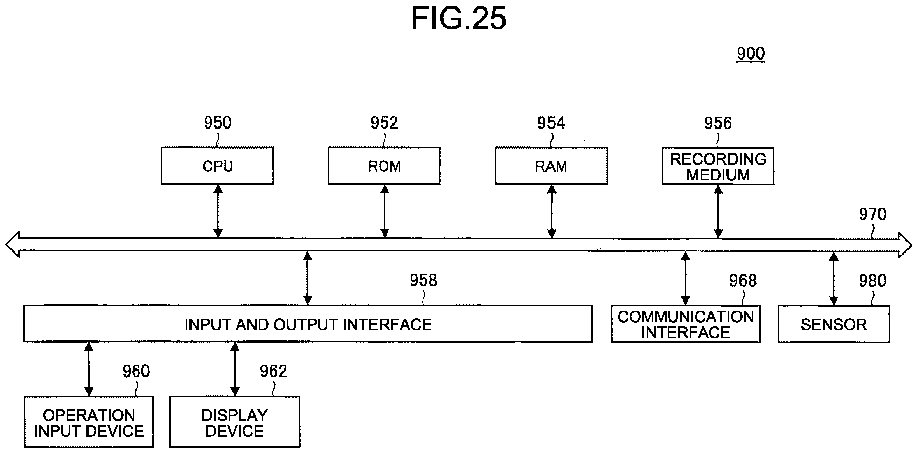

FIG. 25 is an explanatory diagram illustrating an example of a hardware configuration of an information processing device 900 according to this embodiment. In FIG. 25, the information processing device 900 is an example of a hardware component of the server 300 explained above.

The information processing device 900 includes, for example, a CPU 950, a ROM 952, a RAM 954, a recording medium 956, an input and output interface 958, and an operation input device 960. Further, the information processing device 900 includes a display device 962, a communication interface 968, and a sensor 980. In the information processing device 900, the components are connected by, for example, a bus 970 functioning as a data transmission path.

(CPU 950)

The CPU 950 is configured by one or two or more processors configured by an arithmetic circuit such as a CPU, various processing circuits, and the like. The CPU 950 functions as a control unit (not illustrated) that controls the entire information processing device 900 and as the calculating unit 320 explained above.

(ROM 952 and RAM 954)

The ROM 952 stores data for control and the like such as programs and arithmetic parameters to be used by the CPU 950. The RAM 954 temporarily stores, for example, programs to be executed by the CPU 950. The ROM 952 and the RAM 954 play, for example, the function of the storing unit 310 explained above in the information processing device 900.

(Recording medium 956)

The recording medium 956 functions as the storing unit 310 explained above and stores, for example, various data such as data relating to the information processing method according to this embodiment and various applications. Examples of the recording medium 956 include a magnetic recording medium such as a hard disk and a nonvolatile memory such as a flash memory. The recording medium 956 may be detachable from the information processing device 900.

(Input and output interface 958, operation input device 960, and display device 962)

The input and output interface 958 connects, for example, the operation input device 960 and the display device 962. Examples of the input and output interface 958 include a USB (Universal Serial Bus) terminal, a DVI (Digital Visual Interface) terminal, an HDMI (High-Definition Multimedia Interface) (registered trademark) terminal, and various processing circuits.
The operation input device 960 functions as an operation unit (not illustrated) and is included in, for example, the information processing device 900 and connected to the input and output interface 958 on the inside of the information processing device 900. Examples of the operation input device 960 include a keyboard, a button, a direction key, a rotary selector such as a jog dial, a touch panel, or a combination of these devices.
The display device 962 functions as an information presentation device configured by the display device 400 or the like explained above and is provided on, for example, the information processing device 900 and connected to the input and output interface 958 on the inside of the information processing device 900. Examples of the display device 962 include a liquid crystal display and an organic EL display (Organic Electro-Luminescence Display).
Note that it goes without saying that the input and output interface 958 can also be connected to external devices such as an operation input device (for example, a keyboard or a mouse) on the outside and a display device on the outside of the information processing device 900.

(Communication interface 968)

The communication interface 968 is communication means included in the information processing device 900 and functions as the communication unit 308 for performing communication by radio or wire with an external device such as a server via the network 500 explained above (or directly). Examples of the communication interface 968 include a communication antenna and an RF (Radio Frequency) circuit (wireless communication), an IEEE802.15.1 port and a transmission and reception circuit (wireless communication), an IEEE802.11 port and a transmission and reception circuit (wireless communication), or a LAN (Local Area Network) terminal and a transmission and reception circuit (wired communication).

(Sensor 980)

The sensor 980 is a sensor that functions as the biological information sensor 100 explained above and detects, for example, a blood flow signal with any scheme capable of detecting a signal or the like due to a blood flow. The sensor 980 includes, for example, the irradiating unit 102 that emits light and the light receiving unit 104 that generates a signal according to received light. As explained above, the irradiating unit 102 includes one or two or more light sources such as a laser. The light receiving unit 104 includes, for example, a photodiode, an amplifier circuit, a filter circuit, and an analog-digital converter.
The sensor 980 may include, for example, the camera 200 explained above and one or two or more sensors such as an acceleration sensor and a gyro sensor. That is, the sensors included in the sensor 980 are not limited to the example explained above.
Note that the hardware configuration of the information processing device 900 is not limited to the configuration illustrated in FIG. 26. For example, when performing communication with an external device or the like via a connected external communication device or when being configured to perform processing standalone, the information processing device 900 may not include the communication interface 968. The communication interface 968 may have a configuration capable of performing communication with one or two or more external devices according to a plurality of communication schemes. The information processing device 900 can also adopt, for example, a configuration not including the recording medium 956, the operation input device 960, the display device 962, and the like.
This embodiment is explained above with reference to the information processing device 900. However, this embodiment is not limited to such a form. This embodiment can also be applied to various devices capable of performing processing relating to the information processing method according to this embodiment, for example, a communication device such as a cellular phone.

The information processing device 900 according to this embodiment may be applied to a system including a plurality of devices premised on connection to a network (or connection among the devices) like cloud computing. That is, the information processing device 900 according to this embodiment explained above can also be realized as, for example, an information processing system that performs the processing relating to the information processing method according to this embodiment with a plurality of devices.

An example of the hardware configuration of the information processing device 900 is explained above. The components explained above may be configured using general-purpose members or may be configured by hardware specialized for the functions of the components. Such a configuration can be changed as appropriate according to a technical level at each time to be implemented.

<<7. Supplementation>>

Note that the embodiment of the present disclosure explained above can include a program for causing a computer to function as the information processing device according to this embodiment and a non-transitory tangible medium recording the program. The program may be distributed via a communication line (including wireless communication) such as the Internet.

Reference Signs List

10, 10a    INFORMATION PROCESSING SYSTEM

100, 100a          BIOLOGICAL INFORMATION SENSOR
102                IRRADIATING UNIT
104, 104a, 204     LIGHT RECEIVING UNIT
106, 206           CONTROL UNIT
108, 208           COMMUNICATION UNIT
110a, 110b         READOUT UNIT
200                CAMERA
300, 300a, 300b    SERVER
308                COMMUNICATION UNIT
310                STORING UNIT
320, 320a, 320b    CALCULATING UNIT
322                DATA ACQUIRING UNIT
324, 324a          POSITION-INFORMATION CALCULATING UNIT
326                BIOLOGICAL-INFORMATION CALCULATING UNIT
328, 346           INFORMATION OUTPUT UNIT
330                DEPTH-INFORMATION CALCULATING UNIT
332                ACCELERATION ESTIMATING UNIT
340                AUTHENTICATING UNIT
342                COLLATING UNIT
344                DETERMINING UNIT
400                DISPLAY DEVICE
500                NETWORK
700                MEASUREMENT REGION
702                STATIONARY TISSUE
704                MOVING SUBSTANCE
706                PERSON TO BE MEASURED
750                VEHICLE
800                DETECTION SIGNAL
802a, 802b         REGION
804                IMAGE
806                CAPTURED IMAGE
810                WINDOW

| 820, 822 | SECTION |
| 830 | SPECIFIC PART |
| 840 | CHANGE WITH TIME OF BLOOD FLOW INFORMATION |
| 900 | INFORMATION PROCESSING DEVICE |
| 950 | CPU |
| 952 | ROM |
| 954 | RAM |
| 956 | RECORDING MEDIUM |
| 958 | INPUT AND OUTPUT INTERFACE |
| 960 | OPERATION INPUT DEVICE |
| 962 | DISPLAY DEVICE |
| 968 | COMMUNICATION INTERFACE |
| 970 | BUS |
| 980 | SENSOR |

**Claims**

1. An information processing device comprising:

   an irradiating unit (102) configured to irradiate an organism (700) with coherent light;
   a light receiving unit (104) configured to receive the light reflected on the organism (700); and
   a calculating unit (320a) configured to calculate (326), based on sensing data acquired from the light receiving unit, blood flow information linked with position information concerning a position of the reflection of the light in the organism, the position information indicating which region of the organism is included in the sensing data acquired from the light receiving unit, and
   wherein the calculating unit is further configured to calculate (324a) the position information by matching a shape model of the organism with a plurality of kinds of depth information of the organism obtained (330) by processing the sensing data acquired from the light receiving unit and link the calculated position information with the blood flow information obtained by processing the sensing data acquired from the light receiving unit;
   the information processing device being **characterized in that** it comprises
   an estimating unit (332) configured to estimate motion acceleration of the organism based on the plurality of kinds of depth information.

2. The information processing device according to claim 1, wherein the irradiating unit is configured from a laser device.

3. The information processing device according to claim 1, wherein the light receiving unit is provided in noncontact with the organism.

4. The information processing device according to claim 3, wherein the irradiating unit and the light receiving unit are provided in a room or in a vehicle in which the organism is present, and/or
   wherein the light receiving unit is configured from at least any one of a photodiode, a CCD sensor, and a CMOS sensor.

5. The information processing device according to claim 1, wherein

   the light receiving unit includes a plurality of light receiving elements, and
   the calculating unit is configured to link, for each of the light receiving elements, the position information obtained from the sensing data of the light receiving element and the blood flow information, and/or
   wherein the calculating unit is configured to calculate the depth information by calculating a time difference from the irradiation of the light by the irradiating unit until the light reception of the light by the light receiving unit.

6. The information processing device according to claim 1, wherein the calculating unit is configured to calculate the depth information by calculating a phase difference, which is a difference between a phase of the light irradiated by the irradiating unit and a phase of the light received by the light receiving unit.

7. The information processing device according to claim 6, wherein

   the light receiving unit includes first and second readout units (110a, 110b) that are configured to read out, at

# EP 3 915 471 B1

different timings each other, the light received by the light receiving unit, and
the calculating unit is configured to calculate the phase difference based on intensity of the light read out by the first and second readout units.

8. The information processing device according to claim 1, wherein the calculating unit is configured to continuously extract, based on the position information, the blood flow information in a predetermined position set in advance to thereby acquire a change with time of the blood flow information in the predetermined position, and, in particular, wherein the calculating unit is configured to estimate a state of the organism based on the change with time of the blood flow information.

9. The information processing device according to claim 1, wherein the calculating unit is configured to calculate the blood flow information based on an interference light component included in the sensing data or blood alcohol concentration or a blood glucose level based on an absorption spectrum obtained from the sensing data.

10. The information processing device according to claim 9, wherein the calculating unit is configured to calculate, based on the blood flow information, at least any one of a pulse rate, a consumed calorie, a blood pressure, a vascular age, a respiration rate, and an HRV indicator, and/or the information processing device further comprises:

    a collating unit (342) that is configured to perform collation of the organism based on the plurality of kinds of depth information; and
    a determining unit (344) that is configured to determine, based on the blood flow information, whether the depth information is obtained from the organism.

11. An information processing method, comprising:

    irradiating (S301) an organism with coherent light;
    receiving (S307) the light reflected on the organism;
    calculating (S313), based on sensing data based on the received light, blood flow information linked with position information concerning a position of the reflection of the light in the organism, the position information indicating which region of the organism is included in the sensing data acquired from the received light; and
    calculating (S403) the position information by matching a shape model of the organism with a plurality of kinds of depth information of the organism obtained (S315) by processing the sensing data acquired from the received light and linking the calculated position information with the blood flow information obtained by processing the sensing data acquired from received light;
    the method being **characterized in that** it comprises:
    estimating motion acceleration of the organism based on the plurality of kinds of depth information.

12. A computer program comprising instructions which, when the program is executed by a computer, causes the computer to carry out:

    a function (S301) of controlling an irradiating unit to irradiate an organism with coherent light;
    a function (S307) of controlling a light receiving unit to receive the light reflected on the organism; and
    a function (S313) of calculating, based on sensing data acquired from the light receiving unit, blood flow information linked with position information concerning a position of the reflection of the light in the organism, the position information indicating which region of the organism is included in the sensing data acquired from the light receiving unit; and
    a function (S403) of calculating the position information by matching a shape model of an organism with a plurality of kinds of depth information of the organism obtained (S315) by processing the sensing data acquired from the light receiving unit and linking the calculated position information with the blood flow information obtained by processing the sensing data acquired from the light receiving unit;
    the computer program being **characterized in that** it comprises instructions which, when executed by the computer, causes the computer to carry out:
    a function of estimating motion acceleration of the organism based on the plurality of kinds of depth information.

**Patentansprüche**

1. Informationsverarbeitungsvorrichtung, umfassend:

eine Bestrahlungseinheit (102), die konfiguriert ist, um einen Organismus (700) mit kohärentem Licht zu bestrahlen;

eine Lichtempfangseinheit (104), die konfiguriert ist, um das Licht zu empfangen, das auf dem Organismus (700) reflektiert wird; und

eine Berechnungseinheit (320a), die konfiguriert ist, um basierend auf Sensordaten, die von der Lichtempfangseinheit erfasst werden,

Blutflussinformationen zu berechnen (326), die mit Positionsinformationen bezüglich einer Position der Reflexion des Lichts in dem Organismus verknüpft sind, wobei die Positionsinformationen angeben, welcher Bereich des Organismus in den Sensordaten enthalten ist, die von der Lichtempfangseinheit erfasst werden, und

wobei die Berechnungseinheit ferner konfiguriert ist, um die Positionsinformationen durch Abgleichen eines Formmodells des Organismus mit einer Vielzahl von Arten von Tiefeninformationen des Organismus zu berechnen (324a), die durch ein Verarbeiten der Sensordaten erhalten werden (330), die von der Lichtempfangseinheit erfasst werden, und die berechneten Positionsinformationen mit den Blutflussinformationen zu verknüpfen, die durch das Verarbeiten der Sensordaten erhalten werden, die von der Lichtempfangseinheit erfasst werden;

wobei die Informationsverarbeitungsvorrichtung

**dadurch gekennzeichnet ist, dass** sie umfasst

eine Schätzeinheit (332), die konfiguriert ist, um eine Bewegungsbeschleunigung des Organismus basierend auf der Vielzahl von Arten von Tiefeninformationen zu schätzen.

2. Informationsverarbeitungsvorrichtung nach Anspruch 1, wobei die Bestrahlungseinheit aus einer Laservorrichtung konfiguriert ist.

3. Informationsverarbeitungsvorrichtung nach Anspruch 1, wobei die Lichtempfangseinheit berührungslos mit dem Organismus bereitgestellt ist.

4. Informationsverarbeitungsvorrichtung nach Anspruch 3, wobei die Bestrahlungseinheit und die Lichtempfangseinheit in einem Raum oder einem Fahrzeug bereitgestellt sind, in dem sich der Organismus befindet, und/oder wobei die Lichtempfangseinheit aus mindestens einem beliebigen von einer Fotodiode, einem CCD-Sensor und einem CMOS-Sensor konfiguriert ist.

5. Informationsverarbeitungsvorrichtung nach Anspruch 1, wobei

die Lichtempfangseinheit eine Vielzahl von Lichtempfangselementen einschließt, und
die Recheneinheit konfiguriert ist, um für jedes der Lichtempfangselemente die Positionsinformationen, die aus den Sensordaten des Lichtempfangselements erhalten werden, und die Blutflussinformationen zu verknüpfen, und/oder
wobei die Berechnungseinheit konfiguriert ist, um die

Tiefeninformationen durch Berechnen eines Zeitunterschieds von der Bestrahlung mit dem Licht durch die Bestrahlungseinheit bis zu dem Lichtempfang des Lichts durch die Lichtempfangseinheit zu berechnen.

6. Informationsverarbeitungsvorrichtung nach Anspruch 1, wobei die Berechnungseinheit konfiguriert ist, um die Tiefeninformationen durch Berechnen eines Phasenunterschieds zu berechnen, der ein Unterschied zwischen einer Phase des Lichts, das durch die Bestrahlungseinheit ausgestrahlt wird, und einer Phase des Lichts ist, das von der Lichtempfangseinheit empfangen wird.

7. Informationsverarbeitungsvorrichtung nach Anspruch 6, wobei

die Lichtempfangseinheit eine erste und eine zweite Ausleseeinheit (110a, 110b) einschließt, die konfiguriert sind, um das Licht, das durch die Lichtempfangseinheit empfangen wird, zu unterschiedlichen Zeitpunkten auszulesen, und
die Berechnungseinheit konfiguriert ist, um den Phasenunterschied basierend auf einer Intensität des Lichts zu berechnen, das durch die erste und die zweite Ausleseeinheit ausgelesen wird.

8. Informationsverarbeitungsvorrichtung nach Anspruch 1, wobei die Recheneinheit konfiguriert ist, um basierend auf den Positionsinformationen kontinuierlich die Blutflussinformationen an einer zuvor bestimmten Position zu extrahieren, die vorab festgelegt wird, um dadurch eine zeitliche Änderung der Blutflussinformationen an der zuvor bestimmten Position zu erfassen, und insbesondere wobei die Recheneinheit konfiguriert ist, um basierend auf der zeitlichen Änderung der Blutflussinformationen einen Zustand des Organismus zu schätzen.

9. Informationsverarbeitungsvorrichtung nach Anspruch 1, wobei die Berechnungseinheit konfiguriert ist, um die Blutflussinformationen basierend auf einer Interferenzlichtkomponente, die in den Sensordaten eingeschlossen ist, oder einer Blutalkoholkonzentration oder eines Blutzuckerspiegels basierend auf einem Absorptionsspektrums zu berechnen, das aus den Sensordaten erhalten wird.

10. Informationsverarbeitungsvorrichtung nach Anspruch 9, wobei die Berechnungseinheit konfiguriert ist, um basierend auf den Blutflussinformationen mindestens ein beliebiges von einer Pulsfrequenz, einer verbrauchten Kalorie, einem Blutdruck, einem Gefäßalter, einer Atemfrequenz und einem HRV-Indikator zu berechnen, und/oder die Informationsverarbeitungsvorrichtung ferner umfasst:

    eine Vergleichseinheit (342), die konfiguriert ist, um einen Vergleich des Organismus basierend auf der Vielzahl von Arten von Tiefeninformationen durchzuführen; und
    eine Bestimmungseinheit (344), die konfiguriert ist, um basierend auf den Blutflussinformationen zu bestimmen, ob die Tiefeninformationen von dem Organismus erhalten werden.

11. Informationsverarbeitungsverfahren, umfassend:

    Bestrahlen (S301) eines Organismus mit kohärentem Licht;
    Empfangen (S307) des Lichts, das auf dem Organismus reflektiert wird;
    Berechnen (S313), basierend auf Sensordaten basierend auf dem empfangenen Licht, von Blutflussinformationen, die mit Positionsinformationen bezüglich einer Position der Reflexion des Lichts in dem Organismus verknüpft sind, wobei die Positionsinformationen angeben, welcher Bereich des Organismus in den Sensordaten eingeschlossen ist, die von dem empfangenen Licht erhalten werden; und
    Berechnen (S403) der Positionsinformationen durch Abgleichen eines Formmodells des Organismus mit einer Vielzahl von Arten von Tiefeninformationen des Organismus, die durch das Verarbeiten der Sensordaten erhalten werden (S315), die von dem empfangenen Licht erhalten werden, und Verknüpfen der berechneten Positionsinformationen mit den Blutflussinformationen, die durch das Verarbeiten der Sensordaten erhalten werden, die von dem empfangenen Licht erhalten werden;
    wobei das Verfahren **dadurch gekennzeichnet ist, dass** es umfasst:
    Schätzen der Bewegungsbeschleunigung des Organismus basierend auf der Vielzahl von Arten von Tiefeninformationen.

12. Computerprogramm, umfassend Anweisungen, die, wenn das Programm durch einen Computer ausgeführt wird, den Computer veranlassen, um vorzunehmen:

    eine Funktion (S301) zum Steuern einer Bestrahlungseinheit, um einen Organismus mit kohärentem Licht zu bestrahlen;
    eine Funktion (S307) zum Steuern einer Lichtempfangseinheit, um das Licht zu empfangen, das auf dem Organismus reflektiert wird; und
    eine Funktion (S313) zum Berechnen, basierend auf Sensordaten, die von der Lichtempfangseinheit erfasst werden, von Blutflussinformationen, die mit Positionsinformationen bezüglich einer Position der Reflexion des Lichts in dem Organismus verknüpft sind, wobei die Positionsinformationen angeben, welcher Bereich des Organismus in den Sensordaten eingeschlossen ist, die von der Lichtempfangseinheit erfasst werden; und
    eine Funktion (S403) zum Berechnen der Positionsinformationen durch Abgleichen eines Formmodells eines Organismus mit einer Vielzahl von Arten von Tiefeninformationen des Organismus, die durch das Verarbeiten der Sensordaten erhalten werden (S315), die von dem empfangenen Licht erhalten werden, und Verknüpfen der berechneten Positionsinformationen mit den Blutflussinformationen, die durch das Verarbeiten der Sensordaten erhalten werden, die von dem empfangenen Licht erhalten werden;
    wobei das Computerprogramm **dadurch gekennzeichnet ist, dass** es Anweisungen umfasst, die, wenn sie durch den Computer ausgeführt werden, den Computer veranlassen, um vorzunehmen:
    eine Funktion zum Schätzen der Bewegungsbeschleunigung des Organismus basierend auf der Vielzahl von Arten von Tiefeninformationen.

**Revendications**

1. Dispositif de traitement d'informations comprenant :

   une unité d'irradiation (102) configurée pour irradier un organisme (700) avec de la lumière cohérente ;
   une unité de réception de lumière (104) configurée pour recevoir la lumière réfléchie sur l'organisme (700) ; et
   une unité de calcul (320a) configurée pour calculer (326), en fonction de données de captage acquises par l'unité de réception de lumière, des informations de flux sanguin liées à des informations de position concernant une position de la réflexion de la lumière dans l'organisme, les informations de position indiquant quelle région de l'organisme est incluse dans les données de captage acquises par l'unité de réception de lumière, et dans lequel l'unité de calcul est configurée en outre pour calculer (324a) les informations de position en faisant concorder un modèle de forme de l'organisme avec une pluralité de types d'informations de profondeur de l'organisme obtenues (330) par traitement des données de captage acquises par l'unité de réception de lumière et lier les informations de position calculées aux informations de flux sanguin obtenues par traitement des données de captage acquises par l'unité de réception de lumière ;
   le dispositif de traitement d'informations étant **caractérisé en ce qu'**il comprend
   une unité d'estimation (332) configurée pour estimer une accélération de mouvement de l'organisme en fonction de la pluralité de types d'informations de profondeur.

2. Dispositif de traitement d'informations selon la revendication 1, dans lequel l'unité d'irradiation est configurée à partir d'un dispositif laser.

3. Dispositif de traitement d'informations selon la revendication 1, dans lequel l'unité de réception de lumière est fournie sans contact avec l'organisme.

4. Dispositif de traitement d'informations selon la revendication 3, dans lequel l'unité d'irradiation et l'unité de réception de lumière sont fournies dans une pièce ou dans un véhicule dans lequel l'organisme est présent, et/ou dans lequel l'unité de réception de lumière est configurée à partir d'au moins l'une quelconque parmi une photodiode, un capteur CCD et un capteur CMOS.

5. Dispositif de traitement d'informations selon la revendication 1, dans lequel

   l'unité de réception de lumière comporte une pluralité d'éléments de réception de lumière, et
   l'unité de calcul est configurée pour lier, pour chacun des éléments de réception de lumière, les informations de position obtenues à partir des données de captage de l'élément de réception de lumière et les informations de flux sanguin, et/ou
   dans lequel l'unité de calcul est configurée pour calculer les informations de profondeur en calculant une différence de temps allant de l'irradiation de la lumière par l'unité d'irradiation à la réception de lumière de la lumière par l'unité de réception de lumière.

6. Dispositif de traitement d'informations selon la revendication 1, dans lequel l'unité de calcul est configurée pour calculer les informations de profondeur en calculant une différence de phase, qui est une différence entre une phase de la lumière irradiée par l'unité d'irradiation et une phase de la lumière reçue par l'unité de réception de lumière.

7. Dispositif de traitement d'informations selon la revendication 6, dans lequel

   l'unité de réception de lumière comporte des première et seconde unités de lecture (110a, 110b) qui sont configurées pour lire, à des temporisations différentes les unes des autres, la lumière reçue par l'unité de réception de lumière, et
   l'unité de calcul est configurée pour calculer la différence de phase en fonction de l'intensité de la lumière lue par les première et seconde unités de lecture.

8. Dispositif de traitement d'informations selon la revendication 1, dans lequel l'unité de calcul est configurée pour extraire en continu, en fonction des informations de position, les informations de flux sanguin dans une position prédéterminée réglée à l'avance pour acquérir de ce fait un changement au cours du temps des informations de flux sanguin dans la position prédéterminée et, en particulier, dans lequel l'unité de calcul est configurée pour estimer un état de l'organisme en fonction du changement au cours du temps des informations de flux sanguin.

9. Dispositif de traitement d'informations selon la revendication 1, dans lequel l'unité de calcul est configurée pour calculer les informations de flux sanguin en fonction d'une composante de lumière d'interférence incluse dans les données de captage ou un taux d'alcoolémie ou un taux de glycémie en fonction d'un spectre d'absorption obtenu à partir des données de captage.

10. Dispositif de traitement d'informations selon la revendication 9, dans lequel l'unité de calcul est configurée pour calculer, en fonction des informations de flux sanguin, au moins l'un quelconque parmi un pouls, une calorie consommée, une pression sanguine, un âge vasculaire, un rythme respiratoire, et un indicateur VFC, et/ou le dispositif de traitement d'informations comprend en outre :

   une unité de collationnement (342) qui est configurée pour mettre en oeuvre un collationnement de l'organisme en fonction de la pluralité de types d'informations de profondeur ; et
   une unité de détermination (344) qui est configurée pour déterminer, en fonction des informations de flux sanguin, si les informations de profondeur sont obtenues auprès de l'organisme.

11. Procédé de traitement d'informations, comprenant :

   l'irradiation (S301) d'un organisme avec de la lumière cohérente ;
   la réception (S307) de la lumière réfléchie sur l'organisme ;
   le calcul (S313), en fonction de données de captage en fonction de la lumière reçue, d'informations de flux sanguin liées à des informations de position concernant une position de la réflexion de la lumière dans l'organisme, les informations de position indiquant quelle région de l'organisme est incluse dans les données de captage acquises à partir de la lumière reçue ; et
   le calcul (S403) des informations de position en faisant concorder un modèle de forme de l'organisme avec une pluralité de types d'informations de profondeur de l'organisme obtenues (S315) par traitement des données de captage acquises à partir de la lumière reçue et en liant les informations de position calculées aux informations de flux sanguin obtenues par traitement des données de captage acquises à partir de la lumière reçue ;
   le procédé étant **caractérisé en ce qu'**il comprend :
   l'estimation d'une accélération de mouvement de l'organisme en fonction de la pluralité de types d'informations de profondeur.

12. Programme d'ordinateur comprenant des instructions qui, lorsque le programme est exécuté par un ordinateur, amènent l'ordinateur à effectuer :

   une fonction (S301) de commande d'une unité d'irradiation pour irradier un organisme avec de la lumière cohérente ;
   une fonction (S307) de commande d'une unité de réception de lumière pour recevoir la lumière réfléchie sur l'organisme ; et
   une fonction (S313) de calcul, en fonction de données de captage acquises par l'unité de réception de lumière, d'informations de flux sanguin liées à des informations de position concernant une position de la réflexion de la lumière dans l'organisme, les informations de position indiquant quelle région de l'organisme est incluse dans les données de captage acquises par l'unité de réception de lumière ; et
   une fonction (S403) de calcul des informations de position en faisant concorder un modèle de forme d'un organisme avec une pluralité de types d'informations de profondeur de l'organisme obtenues (S315) par traitement des données de captage acquises par l'unité de réception de lumière et en liant les informations de position calculées avec les informations de flux sanguin obtenues par traitement des données de captage acquises par l'unité de réception de lumière ;
   le programme d'ordinateur étant **caractérisé en ce qu'**il comprend des instructions qui, lorsqu'elles sont exécutées par l'ordinateur, amènent l'ordinateur à effectuer :
   une fonction d'estimation d'une accélération de mouvement de l'organisme en fonction de la pluralité de types d'informations de profondeur.

# FIG.1

# FIG.2

SCATTERED LIGHT
(FREQUENCY f) IN
STATIONARY TISSUE

SCATTERED LIGHT
(FREQUENCY f+$\Delta$f) IN
MOVING SUBSTANCE
DOPPLER SHIFT
FREQUENCY

INTERFERENCE

INTERFERENCE
WAVE

BEAT AT
FREQUENCY $\Delta$f

# FIG.3

CHANGE WITH TIME OF
BLOOD FLOW RATE

EP 3 915 471 B1

FIG.4

# FIG.5

<u>10</u>

300

400

500

100

200

BIOLOGICAL
INFORMATION
SENSOR

CAMERA

# FIG.6

100

| | 102 | | 104 |
| IRRADIATING UNIT | | LIGHT RECEIVING UNIT | |

CONTROL UNIT ~106

COMMUNICATION UNIT ~108

# FIG.7

200

LIGHT RECEIVING UNIT ~204

CONTROL UNIT ~206

COMMUNICATION UNIT ~208

# FIG.8

300

CALCULATING UNIT 320

324
POSITION-
INFORMATION
CALCULATING
UNIT

322
DATA
ACQUIRING
UNIT

326
BIOLOGICAL-
INFORMATION
CALCULATING
UNIT

328
INFORMATION
OUTPUT UNIT

310
STORING UNIT

308
COMMUNICA-
TION UNIT

# FIG.9

START

IRRADIATION START OF
LIGHT/IMAGING START ～S101

CALCULATION OF POSITION
INFORMATION ～S103

EXTRACTION OF BLOOD
FLOW INFORMATION OF
SPECIFIC PART ～S105

OUTPUT ～S107

END

# FIG.10

# FIG.11

100a

LIGHT RECEIVING UNIT 104a

IRRADIATING UNIT 102

READOUT UNIT 110a

READOUT UNIT 110b

CONTROL UNIT 106

COMMUNICATION UNIT 108

# FIG.12

IRRADIATION LIGHT

REFLECTED LIGHT

TIME

$q_0$  $q_{90}$  $q_{180}$  $q_{270}$

# FIG.13

IRRADIATION LIGHT

REFLECTED LIGHT

PHASE
DIFFERENCE Φ

READOUT UNIT 110a

802a

READOUT UNIT 110b

802b

# FIG.14

804

Long                    Short

# FIG.15

300a

CALCULATING UNIT 320a

DATA ACQUIRING UNIT 322

DEPTH-INFORMATION CALCULATING UNIT 330

POSITION-INFORMATION CALCULATING UNIT 324a

ACCELERATION ESTIMATING UNIT 332

BIOLOGICAL-INFORMATION CALCULATING UNIT 326

INFORMATION OUTPUT UNIT 328

STORING UNIT 310

COMMUNICA-TION UNIT 308

EP 3 915 471 B1

# FIG.16

START

CARRY OUT SCANNING ~S300

ACQUIRE BLOOD FLOW
INFORMATION OF SPECIFIC
PART ~S400

END

# FIG.17

START

START IRRADIATION OF LIGHT — S301

MEASURE IRRADIATION TIME — S303

SET TO i=0 — S305

SET TO i=i+1 — S321

PERFORM RECEPTION OF REFLECTED LIGHT IN ELEMENT WITH Pixel ID=i — S307

MEASURE LIGHT RECEPTION TIME — S309

CALCULATE POWER SPECTRUM — S311

CALCULATION OF DEPTH INFORMATION — S315

CALCULATION OF BLOOD FLOW INFORMATION — S313

OUTPUT OF BLOOD FLOW INFORMATION — S317

SCANNING IN ALL Pixel ID IS COMPLETED? — S319

NO

YES

END

# FIG.18

```
        ┌─────────────┐
        │    START    │
        └─────────────┘
               │
               ▼
   ┌──────────────────────┐
   │  ACQUISITION OF BLOOD │
   │  FLOW INFORMATION AND │──S401
   │   DEPTH INFORMATION   │
   └──────────────────────┘
               │
               ▼
   ┌──────────────────────┐
   │ CALCULATION OF POSITION│
   │  INFORMATION BASED ON  │──S403
   │   DEPTH INFORMATION    │
   └──────────────────────┘
               │
               ▼
   ┌──────────────────────┐
   │  EXTRACTION OF BLOOD  │
   │  FLOW INFORMATION OF  │──S405
   │     SPECIFIC PART     │
   └──────────────────────┘
               │
               ▼
   ┌──────────────────────┐
   │        OUTPUT         │──S407
   └──────────────────────┘
               │
               ▼
        ┌─────────────┐
        │     END     │
        └─────────────┘
```

# FIG.19

804

830

BLOOD FLOW INFORMATION

840

TIME

Long    Short

EP 3 915 471 B1

# FIG.20

FIG.21

# FIG.22

# FIG.23

300b

CALCULATING UNIT 320b

DATA ACQUIRING UNIT 322

DEPTH-INFORMATION CALCULATING UNIT 330

BIOLOGICAL-INFORMATION CALCULATING UNIT 326

AUTHENTICATING UNIT 340

COLLATING UNIT 342

DETERMIN-ING UNIT 344

INFORMATION OUTPUT UNIT 346

STORING UNIT 310

COMMUNICA-TION UNIT 308

EP 3 915 471 B1

# FIG.24

806

804

AUTHENTICA-
TION OK

BLOOD
FLOW
INFORMA-
TION

840

TIME

# FIG.25

900

| 950 | 952 | 954 | 956 |
|-----|-----|-----|-----|
| CPU | ROM | RAM | RECORDING MEDIUM |

970

958
INPUT AND OUTPUT INTERFACE

968
COMMUNICATION INTERFACE

980
SENSOR

960
OPERATION INPUT DEVICE

962
DISPLAY DEVICE

EP 3 915 471 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018068428 A
- US 20170231544 A1
- US 20180153422 A1